# EUROPEAN PATENT SPECIFICATION

(11) **EP 3 302 297 B1**
(45) Date of publication and mention of the grant of the patent: **20.04.2022**
(21) Application number: 16734021.5
(22) Date of filing: 26.05.2016
(51) Int. Cl.: A61B 17/04, A61F 2/24, A61F 2/86, A61B 17/00, A61B 17/064

(54) **OFF-CENTER TISSUE ANCHORS WITH TENSION MEMBERS**
AUSSERMITTIGE GEWEBEANKER MIT ZUGGLIEDERN
ANCRAGES DE TISSU EXCENTRÉS PRÉSENTANT DES ÉLÉMENTS DE TENSION

(30) Priority: 28.05.2015 US 201562167660 P; 02.12.2015 WO PCT/IB2015/002354
(43) Date of publication of application: 11.04.2018
(73) Proprietor: 4Tech Inc., Waltham, MA 02452-8496 (US)
(72) Inventor: GILMORE, Michael, Ardrahan County Galway (IE); DENTI, Paolo, 20090 Opera (Milano) (IT); LYNN, Kevin, Athenry County Galway (IE); TOBIS, Idan, Beth Hashmonai 99789 (IR); DONNELLY, Evin William, Galway (IE); SOBRINO-SERRANO, Gabriel, Kinvara County Galway H91W 2KX (IE); MULLINS, John, Tuam County Galway (IE); MURPHY, Charlotte, Ardrahan County Galway (IE); GUIDOTTI, Andrea, 8702 Zollikon (CH); VANERMEN, Hugo, 8300 Knocke-le-Zoute (BE); CAMPBELL, Thomas, Westport County Mayo (IE)
(74) Representative: Evens, Paul Jonathan
(86) International application number: PCT/IB2016/000840
(87) International publication number: WO 2016/189391

(56) References cited:
- WO-A1-2011/089601
- WO-A1-2013/003228
- WO-A1-2015/015497
- WO-A1-2015/015497
- WO-A1-2015/015497
- US-A1- 2002 013 571
- US-A1- 2005 251 208
- US-A1- 2005 251 208
- US-A1- 2005 251 208
- US-A1- 2007 185 532
- US-A1- 2008 027 446
- US-A1- 2008 027 446
- US-A1- 2008 027 446

## Description

### FIELD OF THE INVENTION

The present invention relates generally to tissue anchors, and specifically to tissue anchors for implantation in soft tissue, such as cardiac tissue.

### BACKGROUND

Tissue anchors are used for anchoring elements, such as leads or sutures, to tissue, such as bone or soft tissue. US 2005/0251208 A1 shows examples of such tissue anchors, and specifically a tissue anchor (depicted in figures 33A and 33B of this prior art document) adapted to be delivered in a constrained state within a deployment tool through a tissue wall, the tissue anchor comprising: a protective sleeve having a central longitudinal axis; a metallic basket, configured to have a predetermined shape when unconstrained by the deployment tool and automatically transition to the predetermined shape when released from being constrained by the deployment tool, and which (a) extends proximally from a distal end of the protective sleeve, and (b) comprises struts, wherein when the tissue anchor is unconstrained by the deployment tool: (a) the struts are shaped as an open shape, and (b) if the tissue-coupling element were to be projected onto a plane that is perpendicular to the central longitudinal axis (as depicted in figure 33B), the projection of the struts would form sectors of about 60 degrees; and a suture, which includes (a) a distal portion that is fixed to a distal collar on a distal site on the struts, (b) a proximal portion, which has a longitudinal segment that runs along the inside of the protective sleeve, and thus crosses the open shape from the distal end of the open shape to the proximal end of the open shape along the inside of the protective sleeve and beyond when the tissue anchor is unconstrained by the deployment tool.

### SUMMARY OF THE INVENTION

The invention is as defined in the appended set of claims.

The present invention will be more fully understood from the following detailed description of embodiments thereof, taken together with the drawings, in which:

### BRIEF DESCRIPTION OF THE DRAWINGS

Fig. 1A is a schematic illustration of a tissue anchor before deployment from a deployment tool, in accordance with an application of the present invention;
Figs. 1B-D are schematic illustrations of the tissue anchor of Fig. 1A after deployment from the deployment tool;
Figs. 2A-B are schematic illustrations of another tissue anchor before deployment from a deployment tool;
Figs. 3A-C are schematic illustrations of the tissue anchor of Figs. 2A-B after deployment from the deployment tool;
Fig. 3D is a schematic illustration of an anchor head of the tissue anchor of Figs. 2A-3C;
Figs. 4A-B are schematic illustrations of two configurations of a tissue anchor system;
Figs. 5A-B are schematic illustrations of a tissue-coupling element and an anchor shaft of the tissue anchor of Figs. 2A-3C;
Figs. 5C-D are schematic illustrations of a tissue-coupling element and an anchor shaft of the tissue anchor of Figs. 2A-3C;
Figs. 6A-B are schematic illustrations of yet another tissue anchor before deployment from a deployment tool;
Fig. 7 is a schematic illustration of still another tissue anchor after deployment from a deployment tool;
Fig. 8 is a schematic illustration of another tissue anchor after deployment from a deployment tool;
Figs. 9A-F are schematic illustrations of alternative ways to fix a flexible elongate tension member to a site of an open loop of a tissue anchor;
Figs. 10A, B, H are schematic illustrations of configurations of tissue anchors, in accordance with respective applications of the present invention;
Figs. 10C-G are schematic illustrations of configurations of other tissue anchors; Fig. 11A is a schematic illustration of the tissue anchor of Fig. 10E before deployment from a deployment tool;
Fig. 11B is a schematic illustration of the tissue anchor of Fig. 10F before deployment from a deployment tool;
Fig. 12 is a schematic illustration of another configuration of an open loop of a tissue anchor;
Figs. 13A-D are schematic illustrations of another tissue anchor having a locking stopper;
Fig. 13E is a schematic illustration of the tissue anchor of Figs. 13A-D comprising a sealing element;
Figs. 14A-D are schematic illustrations of a method for deploying the tissue anchor system of Fig. 4B for repairing a tricuspid valve;
Figs. 15A-C are schematic illustrations of a method for deploying the tissue anchor system of Fig. 4A for repairing the tricuspid valve; and
Fig. 16 is a schematic illustration of several external exit sites.

### DETAILED DESCRIPTION

Some embodiments of the present invention provide a tissue anchor 20 and a deployment tool 30, which is typically configured to deliver the tissue anchor through a wall of a heart of a subject.

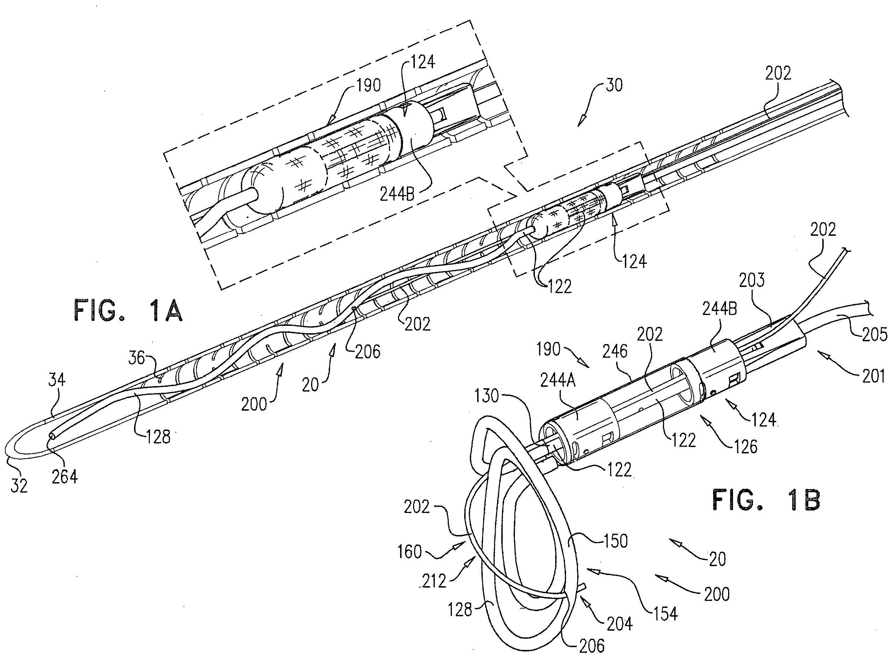

Fig. 1A is a schematic illustration of a tissue anchor 200 before deployment from deployment tool 30, and Figs. 1B-D are schematic illustrations of tissue anchor 200 after deployment from deployment tool 30.

Tissue anchor 200 is one implementation of tissue anchor 20, described above. Tissue anchor 200 comprises (a) an anchor shaft 122, (b) an anchor head 124 connected to a proximal portion 126 of anchor shaft 122, and (c) a tissue-coupling element 128, which extends from a distal end 130 of anchor shaft 122. For some applications, anchor shaft 122 and tissue-coupling element 128 are integral to one another; for example, anchor shaft 122 and tissue-coupling element 128 may comprise a wire.

Deployment tool 30 is configured to constrain tissue-coupling element 128 while delivering tissue-coupling element 128 through tissue. For some applications, deployment tool 30 is shaped so as to define a sharp distal piercing tip 32, which is advanced through the wall of the heart of the subject. Typically, during delivery, such as shown in Fig. 1A, deployment tool 30 is configured to hold tissue-coupling element 128 in an elongated, unwound configuration, which may be straight (configuration not shown) or curvy (such as shown in Fig. 1A). For some applications, deployment tool 30 comprises a deployment shaft 34 shaped so as to define a deployment-shaft lumen, such as a hypodermic needle. The deployment-shaft lumen is sized to hold tissue-coupling element 128 constrained therein, and, optionally, to hold other portions of tissue anchor 20 therein, such as anchor shaft 122 and/or anchor head 124. For some applications, deployment tool 30 has a length of between 100 and 180 cm, and/or an inner diameter of between 2 and 6 mm. For some applications, deployment tool 30 comprises a distal-most rigid portion, which typically has a length of 5 to 25 mm, and the remaining proximal portion of the deployment tool is flexible (but not extendable or compressible). For some applications, the proximal portion is shaped so as to define one or more lateral slots 36, which provide flexibility to the proximal portion, while maintaining a backbone that prevents longitudinal compression and extension of the proximal portion. Typically, deployment tool 30 is advanced within a steerable catheter tube, as is known in the art, which may, for example, comprise a braided material. Typically, tissue anchor 20 is provided in sterile packaging, optionally pre-positioned in deployment tool 30.

For some applications, tissue-coupling element 128 comprises a wire 150. For some applications, a cross-sectional area of wire 150 is at least 0.09 mm2 (such as at least 0.18 mm2), no more than 3 mm2 (e.g., no more than 2.9 mm2), and/or between 0.09 mm2 (such as 0.18 mm2) and 3 mm2 (e.g., 2.9 mm2). For some applications, wire 150 has a circular cross-section, and a diameter of wire 150 is at least 0.18 mm, no more than 2 mm, and/or between 0.18 and 2 mm. For some applications, a distal end 152 of wire 150 does not define a sharp distal tip; for example, the distal end may be blunt. For some applications, wire 150 comprises metal, such as Nitinol. For some applications, wire 150 comprises one or more radiopaque markers.

Fig. 1A shows tissue anchor 200 (including tissue-coupling element 128, anchor shaft 122, and anchor head 124) fully constrained by deployment tool 30. Typically, when tissue-coupling element 128 is constrained by the deployment tool, a longitudinal portion of flexible elongate tension member 202, described hereinbelow, runs alongside a portion of wire 150. For some applications, deployment tool 30 comprises a removable driver 201, which comprises a driver head 203 and at least one shaft 205 that is coupled to the driver head. Driver head 203 is removably coupled to anchor head 124 during penetration of tissue-coupling element 128 through tissue, as described hereinbelow. The at least one shaft 205 is configured to controllably detach the driver head from the anchor head. For example, a deployment needle may run through a channel of the at least one shaft; pulling on the needle detaches the driver head from the anchor head.

When tissue anchor 200 is fully constrained by deployment tool 30, tissue-coupling element 128 typically has an outer diameter of at least 0.3 mm, no more than 4 mm, and/or between 0.3 and 4 mm, such as at least 1 mm, no more than 3 mm, and/or between 1 and 3 mm.

For some applications, anchor shaft 122 and tissue-coupling element 128 are integral to one another; for example, anchor shaft 122 and tissue-coupling element 128 may both comprise wire 150, as shown.

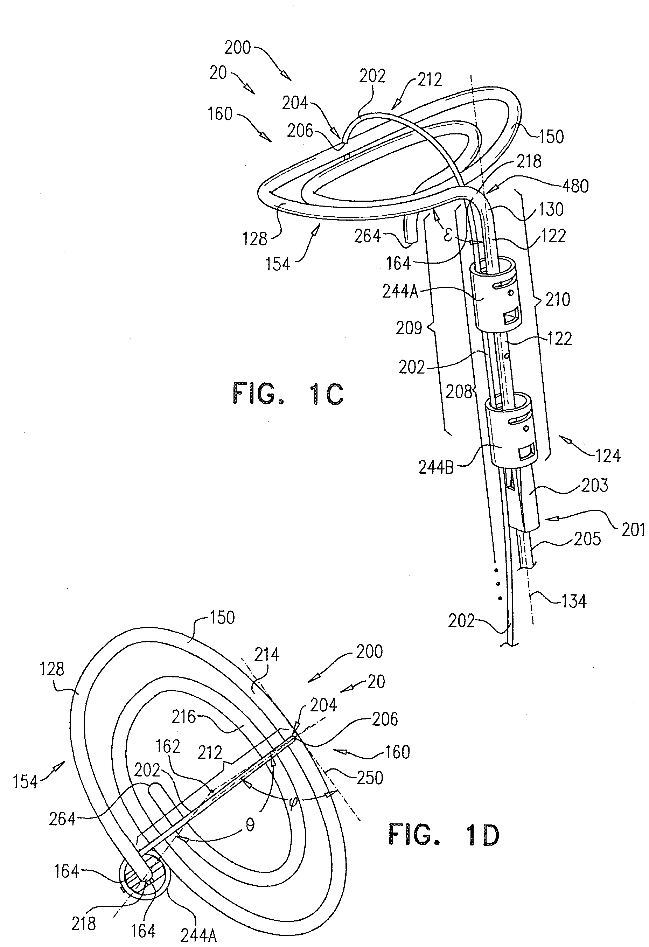

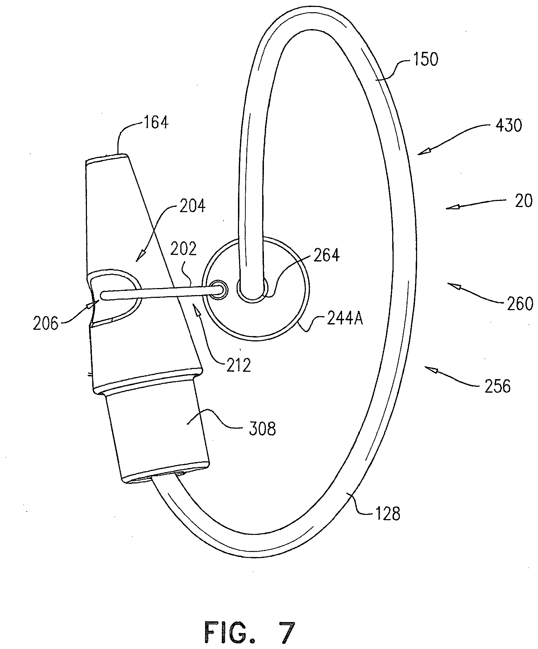

When tissue anchor 200 is unconstrained by deployment tool 30, such as shown in Figs. 1B-D, wire 150 is shaped as an open loop 154 (e.g., a three-dimensional open loop), such as a spiral 160 (e.g., a three-dimensional spiral) around a center point 162 (labeled in Fig. 1D). For some applications, such as shown in Figs. 1B-D, wire 150 extends from distal end 130 of anchor shaft 122 at a radially-outer end 164 of open loop 154 (e.g., spiral 160) (labeled in Figs. 1C and 1D), when tissue anchor 200 is unconstrained by deployment tool 30. For some applications, wire 150 intersects center point 162 when tissue anchor 200 is unconstrained by deployment tool 30 (configuration not shown), while for other applications, wire 150 does not intersect center point 162 when tissue anchor 200 is unconstrained by deployment tool 30 (as shown). For other applications, such as shown in Fig. 7, described hereinbelow, wire 150 extends from distal end 130 of anchor shaft 122 at radially-inner end 264 of an open loop 354 (e.g., a spiral 360).

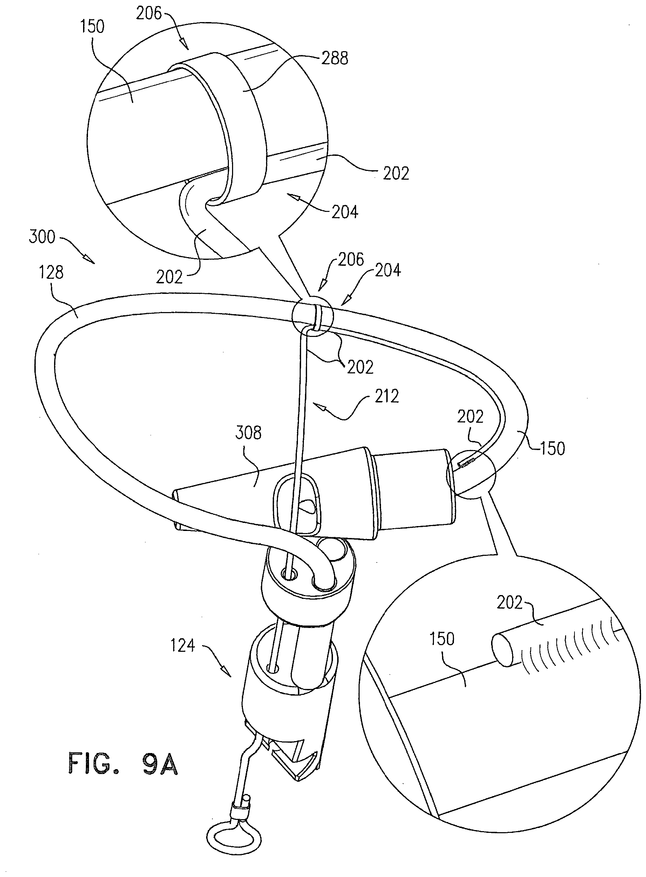

Tissue anchor 200 further comprises a flexible elongate tension member 202, which includes:
- a distal portion 204 that is fixed to a site 206 on open loop 154 (e.g., spiral 160) (such as by welding, soldering, crimping, and/or knotting, and/or as described hereinbelow with reference to Figs. 9A-F),
- a proximal portion 208, which has a longitudinal segment 209 that runs alongside at least a portion 210 of anchor shaft 122 (labeled in Fig. 1C, in which the at least a portion 210 of anchor shaft 122 is the entire length of anchor shaft 122), and
- a crossing portion 212, which (a) is disposed between distal and proximal portions 204 and 208 along flexible elongate tension member 202, and (b) crosses at least a portion of open loop 154 (e.g., spiral 160) when tissue anchor 200 is unconstrained by deployment tool 30.

Although flexible elongate tension member 202 is fixed to wire 150 of tissue-coupling element 128, flexible elongate tension member 202 is typically distinct from wire 150. In other words, flexible elongate tension member 202 and wire 150 are not two longitudinal portions of a single continuous wire, i.e., are not longitudinally contiguous with each other.

Tension is applied to tissue-coupling element 128 of tissue anchor 200 via flexible elongate tension member 202. The applied tension is resisted by the outward force of open loop 154 (e.g., spiral 160). The applied tension at least partially compresses and stiffens open loop 154 (e.g., spiral 160). This arrangement of tension distribution may overcome any natural tendency of open loop 154 (e.g., spiral 160) to straighten (i.e., unwind) if tension were to be applied along a central longitudinal axis 134 of anchor shaft 122 via anchor shaft 122, and thus may allow the application of a greater load to open loop 154 (e.g., spiral 160). In addition, this stiffening technique allows open loop 154 (e.g., spiral 160) to be manufactured less stiff than it otherwise would need to be, which facilitates straightening and delivering the tissue anchor, and subsequent stiffening *in situ.*

Typically, before tension is applied to flexible elongate tension member 202, when tissue anchor 200 is unconstrained by deployment tool 30, flexible elongate tension member 202 is not taut across the at least a portion of open loop 154 (e.g., spiral 160). For example, flexible elongate tension member 202 may arc distally, such as can best be seen in Fig. 1C.

Typically, tissue anchor 200 is configured to allow relative axial motion between the at least a portion 210 of anchor shaft 122 and longitudinal segment 209 of proximal portion 208 of flexible elongate tension member 202 when tissue anchor 200 is unconstrained by deployment tool 30 (as flexible elongate tension member 202 is tensioned and pulls on tissue-coupling element 128, tissue anchor 200 becomes progressively more constrained by flexible elongate tension member 202; the relative axial motion nevertheless remains possible). In other words, longitudinal segment 209 of proximal portion 208 of flexible elongate tension member 202 is axially moveable with respect to the at least a portion 210 of anchor shaft 122 when tissue anchor 200 is unconstrained by deployment tool 30. Such axial motion allows tension to be applied to flexible elongate tension member 202 without also being applied to anchor shaft 122, and allows open loop 154 (e.g., spiral 160) to be unwound and flexible elongate tension member 202 to be disposed alongside a portion of flexible elongate tension member 202, as shown in Fig. 1A (in which deployment tool 30 constrains both constrain tissue-coupling element 128 and flexible elongate tension member 202). Typically, longitudinal segment 209 of proximal portion 208 of flexible elongate tension member 202 is coupled in sliding communication with the at least a portion 210 of anchor shaft 122, when tissue anchor 200 is unconstrained by deployment tool 30. For some applications, tissue anchor 200 comprises one or more annular elements, which are disposed around the at least a portion of anchor shaft 122, and couple flexible elongate tension member 202 in the sliding communication with the at least a portion 210 of anchor shaft 122, when tissue anchor 200 is unconstrained by deployment tool 30. For example, the annular elements may comprise one or more collars 244, described hereinbelow, loops, or rings.

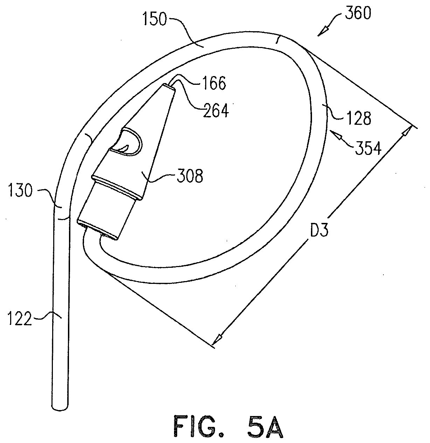

For some applications, flexible elongate tension member 202 is not fixed to any portion of open loop 154 (e.g., spiral 160) beyond 2 mm from site 206 on open loop 154 (e.g., spiral 160), measured when tissue anchor 200 is unconstrained by deployment tool 30. Alternatively or additionally, when tissue anchor 200 is unconstrained by deployment tool 30, flexible elongate tension member 202 is not fixed to any portion of open loop 154 (e.g., spiral 160) beyond a distance from site 206 on open loop 154 (e.g., spiral 160), which distance equals 30% of greatest lateral dimension D3 of open loop 154 (e.g., spiral 160) of tissue-coupling element 128, measured perpendicular to central longitudinal axis 134 (labeled in Fig. 5A). For some applications, flexible elongate tension member 202 is fixed to open loop 154 (e.g., spiral 160) only at site 206 on open loop 154 (e.g., spiral 160). Alternatively, a distal portion of flexible elongate tension member 202 beyond site 206 is fixed to open loop 154 (e.g., spiral 160), such as described hereinbelow with reference to Figs. 9A-E.

Typically, when tissue anchor 200 is unconstrained by deployment tool 30, the at least a portion of open loop 154 (e.g., spiral 160) crossed by crossing portion 212 has a length that equals at least 33% (e.g., at least 50%, at least 75%, or at least 90%) of greatest lateral dimension of open loop 154 (e.g., spiral 160) of tissue-coupling element 128, measured perpendicular to central longitudinal axis 134. (A similar greatest lateral dimension D3 of open loop 354 of tissue anchor 300 is labeled in Fig. 5A.)

For some applications, as shown, site 206 is on an outermost turn 214 of open loop 154 (e.g., spiral 160) (labeled in Fig. 1D), when tissue anchor 200 is unconstrained by deployment tool 30. For some other applications, site 206 is on a second-to-outermost turn 216 of open loop 154 (e.g., spiral 160) (labeled in Fig. 1D), when tissue anchor 200 is unconstrained by deployment tool 30 (configuration not shown).

Typically, a radius of flexible elongate tension member 202 is less than a radius of wire 150, such as less than 50% of the radius of wire 150. For some applications a cross-sectional area of wire 150 is at least 0.09 mm2 (such as at least 0.18 mm2), no more than 3 mm2 (e.g., no more than 2.9 mm2), and/or between 0.09 mm2 (such as 0.18 mm2) and 3 mm2 (e.g., 2.9 mm2). For some applications, flexible elongate tension member 202 comprises metal, such as a metal alloy, e.g., Nitinol. For some applications, flexible elongate tension member 202 comprises radiopaque sections or is radiopaque, to enable observation of the relative movement when tensioning.

For some applications, site 206 on open loop 154 (e.g., spiral 160) is a first site 206 on open loop 154 (e.g., spiral 160), and, when tissue anchor 200 is unconstrained by deployment tool 30 and flexible elongate tension member 202 is tensioned straight, (a) wire 150 extends from distal end 130 of anchor shaft 122 at a second site 218 on open loop 154 (e.g., spiral 160), and (b) if tissue-coupling element 128 and flexible elongate tension member 202 were to be projected onto plane 136 that is perpendicular to central longitudinal axis 134, an angle Θ (theta) between the first and the second sites, having a vertex 242 at center point 162, would be between 130 and 180 degrees, such as between 150 and 180 degrees, e.g., between 170 and 180 degrees (labeled in Fig. 1D). For some applications, as shown, second site 218 is at radially-outer end 164 of open loop 154 (e.g., spiral 160).

Alternatively or additionally, for some applications, as labeled in Fig. 1D, when tissue anchor 200 is unconstrained by deployment tool 30 and flexible elongate tension member 202 is tensioned straight, if tissue-coupling element 128 and flexible elongate tension member 202 were to be projected onto plane 136 that is perpendicular to central longitudinal axis 134, an angle ϕ (phi) between (a) flexible elongate tension member 202 and (b) a tangent 250 to open loop 154 (e.g., spiral 160) at site 206 would be between 45 and 90 degrees, such as between 70 and 90 degrees, e.g., 90 degrees.

For some application, anchor shaft 122 comprises a sealing element 190. For some applications, sealing element 190 comprises one or more collars 244 disposed around anchor shaft 122, and, typically, a sleeve 246 that couples the collars 244 together. Sleeve 246 defines a lumen having proximal and distal ends. The flexible elongate tension member 202 slidingly passes through the lumen and its ends. (Sleeve 246 is shown in Fig. 1A; for clarity of illustration, sleeve 246 is shown as transparent in Fig. 1B, and is not shown in Fig. 1C.) In this configuration, sealing element 190 is typically sized and shaped to be inserted into the incision through the heart wall, and to provide a blood-tight seal. Sleeve 246, if provided, occludes blood flow to provide the seal. For some applications, sleeve 246 promotes hemostasis. Optionally, filament or fiber is provided within sleeve 246 to promote hemostasis. For example, sleeve 246 may comprise Dacron, and/or may be coated and/or woven to facilitate clotting.

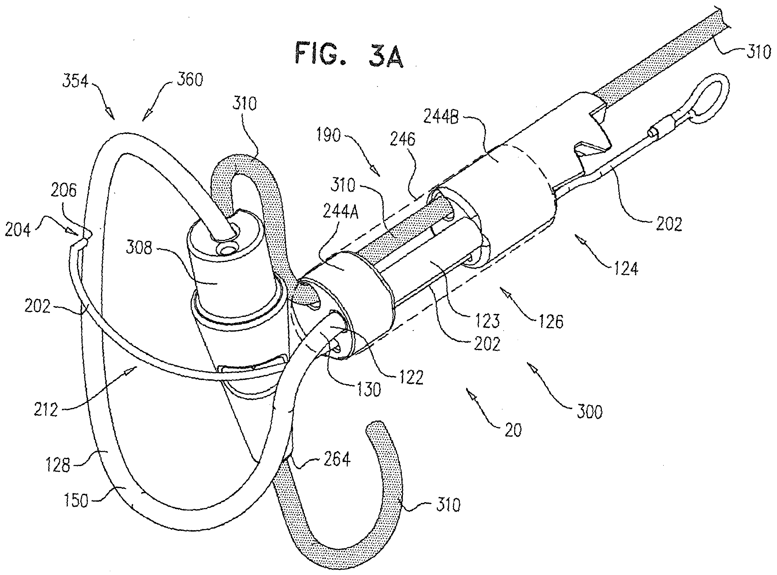

For some applications, collars 244 comprise a distal guide collar 244A and a proximal driver collar 244B, which optionally are components of or serve as anchor head 124. For some applications, a proximal end of anchor shaft 122 is fixed within proximal driver collar 244B, as shown in Fig. 1C, or within distal driver collar 244A, as shown in Fig. 3A for tissue anchor 300. For some applications, one or more of collars 244 are radiopaque or comprise a radiopaque marker. For some applications, proximal driver collar 244B is shaped so as to be removably coupleable with an anchor-deployment shaft, such as described regarding a helical tissue anchor in PCT Publication WO 2015/063580.

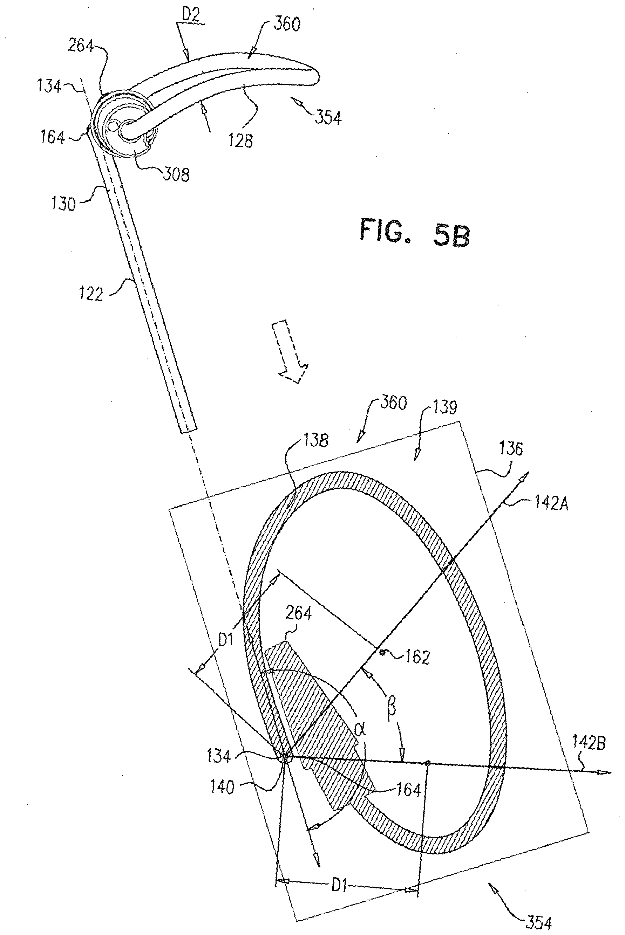

For some applications, a proximally-facing surface defined by tissue-coupling element 128 is convex when tissue anchor 200 is unconstrained by deployment tool 30, such as shown in Figs. 1B and 1C. For other applications, a proximally-facing surface defined by tissue-coupling element 128 is concave when tissue anchor 200 is unconstrained by deployment tool 30, such as shown in Figs. 5B for tissue anchor 300.

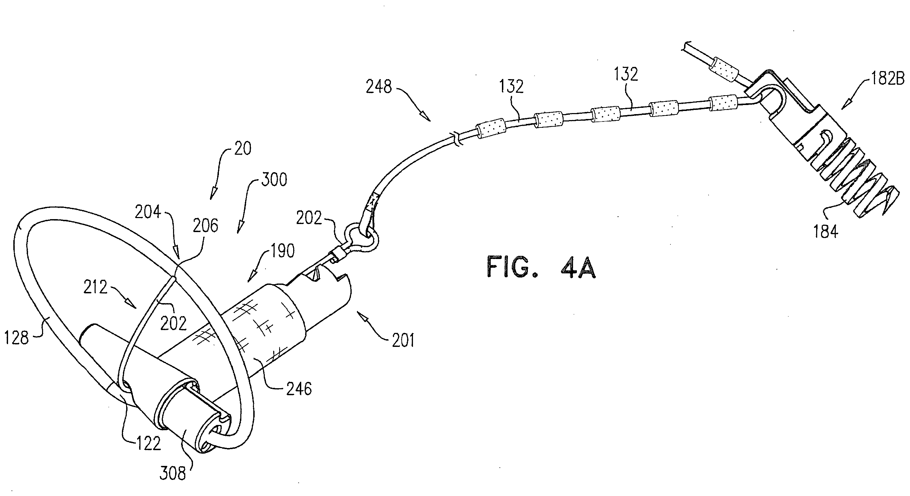

For some applications, such as shown in Figs. 4A-B for tissue anchor 300 (described hereinbelow), one or more tethers 132 are provided, which are configured to be coupled to tissue anchor 200. Typically, the one or more tethers 132 are fixed to flexible elongate tension member 202, typically to proximal portion 208 of the tension member, such as at or near (e.g., within 1 cm of) a proximal end of proximal portion 208. When tension is applied to the one or more tethers, the tension is transmitted to flexible elongate tension member 202, rather than to anchor shaft 122 via anchor head 124. Typically, the one or more tethers are (a) fixed to the second tissue anchor and (b) not fixed to anchor shaft 122 of first tissue anchor 200.

For some applications, radially-inner end 264 of open loop 154 (e.g., spiral 160) is bent proximally, such as can be best seen in Fig. 1C. Because of the bend, radially-inner end 264 may help tissue-coupling element 128 resist rotation and uncoiling.

For some applications, when tissue anchor 200 is unconstrained by deployment tool 30, such as shown in Figs. 1B-D, wire 150 (a) is shaped as an open loop 154 having more than one turn, such that a first complete turn of open loop 154 at least partially overlaps (i.e., runs alongside, above, and/or below) a second at-least-partial turn of open loop 154. For some applications, the first complete turn and the second at-least-partial turn radially coincide, i.e., are at a same distance as each other from a center point (configuration not shown). For other applications, as shown in the figures, an outermost turn of open loop 154 at least partially overlaps (i.e., runs alongside, above, and/or below) a second-to-outermost turn of open loop 154 (for example, an outermost turn 214 and a second-to-outermost turn 216 of open loop 154 are labeled in Fig. 1D). (As used herein, one turn equals 360 degrees. As used herein, "more than one turn" should not be understood as requiring at least two turns; instead, "more than one turn" also includes one turn plus a fraction of a turn, as described below. For example, for applications in which open loop 154 includes an outermost turn and a second-to-outermost turn, the second-to-outermost turn of open loop 154 may be a partial turn, such as shown in Figs. 3A-C, 4A-B, 5A-D, 7, 8, 9A-F, 10E, 10G, 12, and 13A-E.)

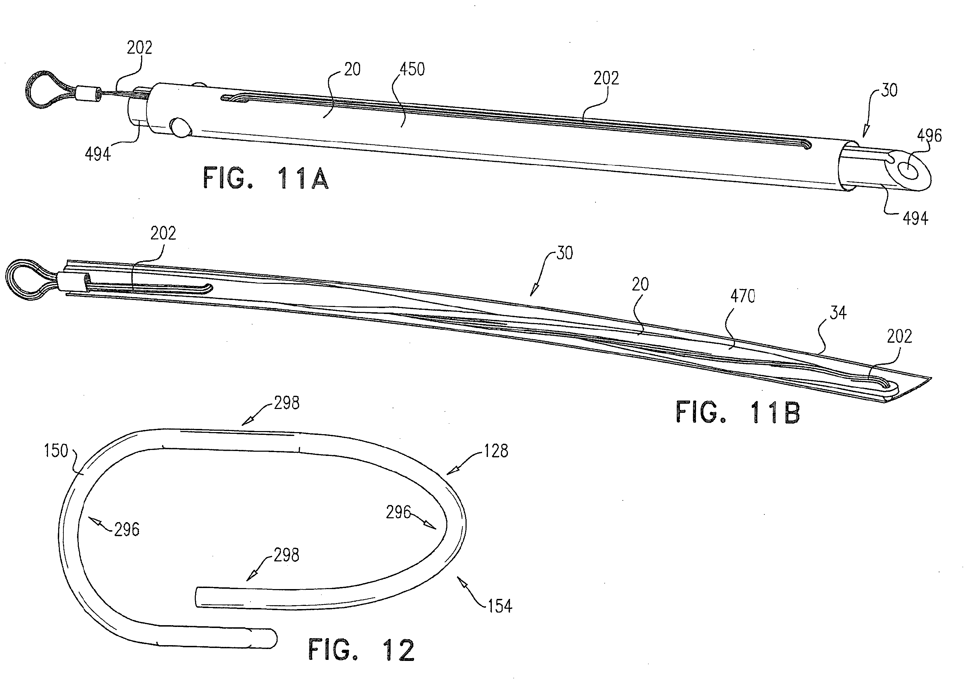

For applications in which open loop 154 includes an outermost turn and a second-to-outermost turn, such as shown in Fig. 1D, open loop 154 has radially-outer end 164 and radially-inner end 264, which typically do not touch each other at least when tissue anchor 200 is unconstrained by deployment tool 30. For applications in which the first complete turn and the second at-least-partial turn radially coincide, the two opposite ends of the open loop typically do not touch each other at least when tissue anchor 200 is unconstrained by deployment tool 30. Open loop 154 is defined by an elongate path of wire 150 that winds more than one turn around center point 162 without forming a closed loop. The elongate path may include one or more curved segments and/or one or more straight segments, such as described hereinbelow with reference to Fig. 12. The path may fall in two dimensions, or may fall in three dimensions, in which case the open loop is a three-dimensional open loop, the elongate path of which winds around a center axis while moving parallel to the axis, without forming a closed loop.

For some applications, open loop 154 extends from distal end 130 of anchor shaft 122 at radially-outer end 164 of open loop 154. For some applications, wire 150 intersects center point 162 (labeled in Fig. 1D) when tissue anchor 200 is unconstrained by deployment tool 30 (configuration not shown), while for other applications, wire 150 does not intersect center point 162 (labeled in Fig. 1D) when tissue anchor 200 is unconstrained by deployment tool 30 (as shown).

For some applications, when tissue anchor 200 is unconstrained by deployment tool 30, such as shown in Figs. 1B-D, wire 150 of open loop 154 is shaped as a spiral 160 (e.g., a three-dimensional spiral) around center point 162. For some of these applications, wire 150 of spiral 160 extends from distal end 130 of anchor shaft 122 at radially-outer end 164 of spiral 160. For some applications, wire 150 of spiral 160 intersects center point 162 when tissue anchor 200 is unconstrained by deployment tool 30 (configuration not shown), while for other applications, wire 150 of spiral 160 does not intersect center point 162 when tissue anchor 200 is unconstrained by deployment tool 30 (as shown). For some applications, spiral 160 is generally circular when tissue anchor 200 is unconstrained by deployment tool 30 (configuration not shown), while for other applications, spiral 160 is an elliptical spiral when the tissue anchor is unconstrained by deployment tool 30, such as shown in Figs. 1B-D.

As used herein, center point 162 is the centroid of projection 139 of tissue-coupling element 128 on plane 136. Typically, such as when tissue-coupling element 128 is shaped as a spiral, tissue-coupling element 128 is non-helical when tissue anchor 200 is unconstrained by deployment tool 30.

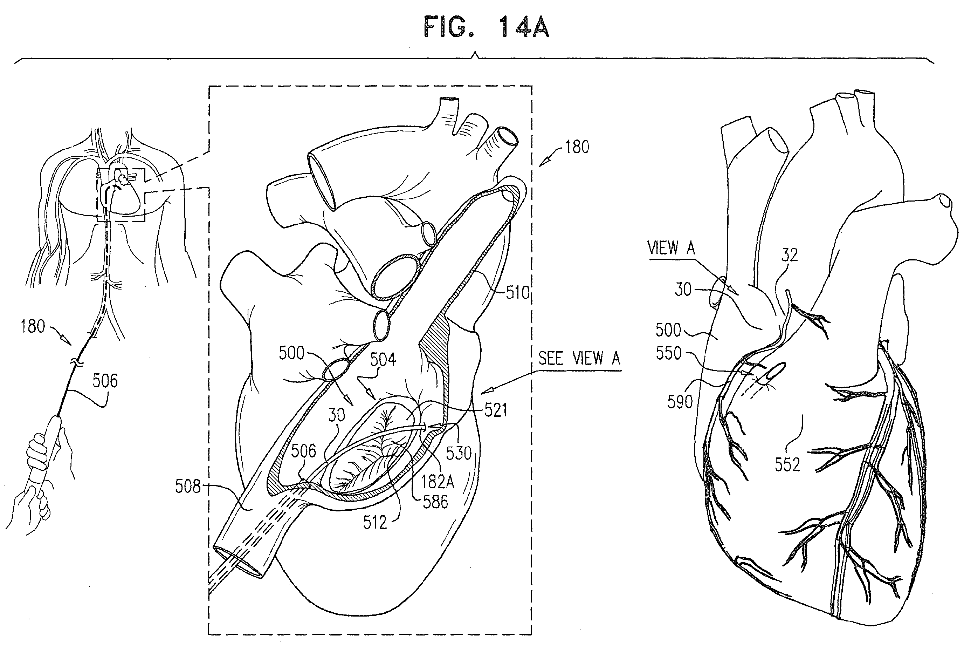

Reference is made to Figs. 1A-D. For some applications, tissue anchor 200 is implanted using techniques described hereinbelow with reference to Figs. 14A-D, 15A-C, and/or 16, optionally in combination with techniques described in one or more of the patents and patent application publications referenced below, *mutatis mutandis.*

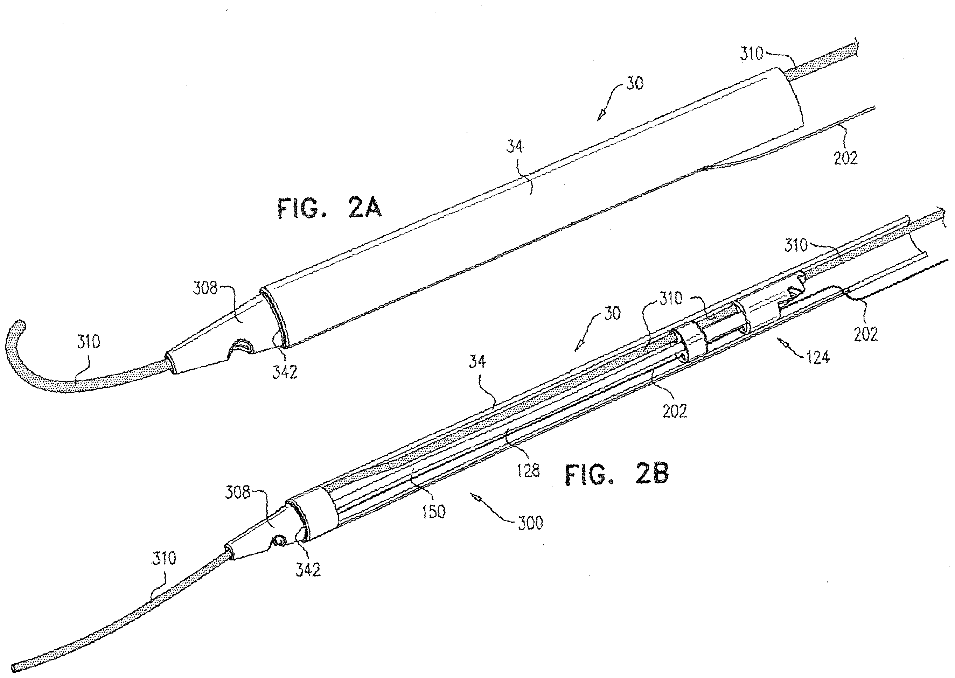

Reference is now made to Figs. 2A-B and 3A-C, which are schematic illustrations of a tissue anchor 300 before (Figs. 2A-B) and after (Figs. 3A-C) deployment from deployment tool 30. Tissue anchor 300 is one implementation of tissue anchor 20, described above. Other than as described below and shown in the figures, tissue anchor 300 is generally similar to tissue anchor 200, described hereinabove with reference to Figs. 1A-D, and may implement any of the features thereof, *mutatis mutandis.*

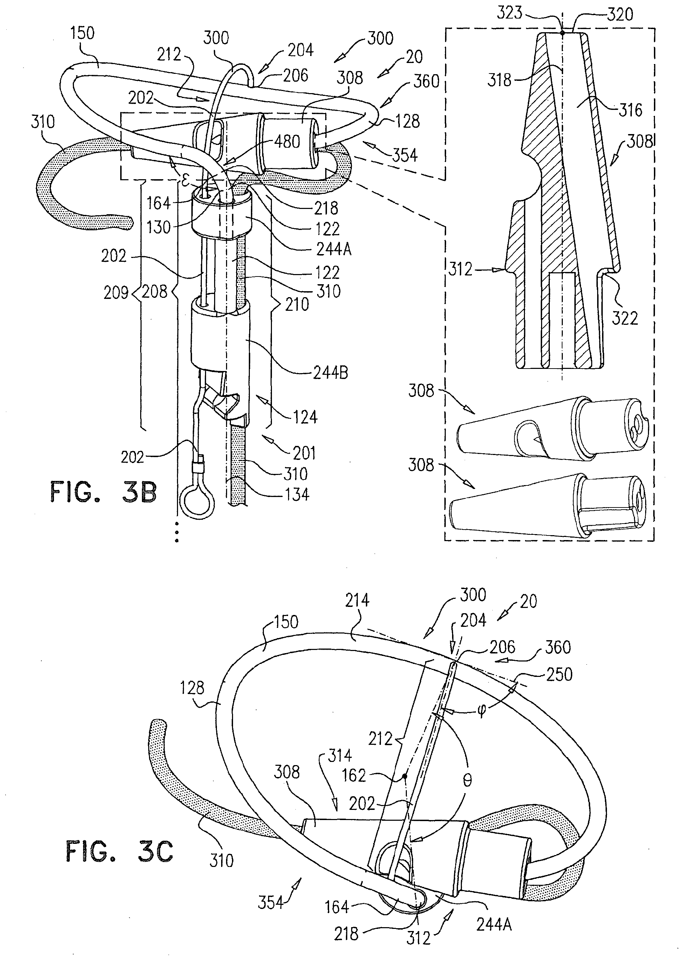

For some applications, tissue-coupling element 128 comprises a tip 308, which is fixed to a distal end of wire 150, and has, at a widest longitudinal site 312 along tip 308 (labeled in Fig. 3C), a greatest tip outer cross-sectional area that equals at least 150% (e.g., at least 200%, or at least 300%) of an average wire cross-sectional area of wire 150 (The cross-sectional area of tip 308 is measured perpendicular to a central longitudinal axis 318 of tip 308. Similarly, the cross-sectional area of wire 150 is measured perpendicular to a central longitudinal axis of the wire, and is not a cross-sectional area of open loop 154.) Alternatively or additionally, for some applications, the greatest tip outer cross-sectional area is greater than 1 mm2, e.g., greater than 1.5 mm2, such as greater than 2 mm2. For some applications, tip 308 includes a frustoconical portion 314 (labeled in Fig. 3C).

Typically, tip 308 is shaped so as to define a guidewire lumen 316 therethrough. For some applications, central longitudinal axis 318 of tip 308 (a) passes through a distal end-opening 320 of guidewire lumen 316, and (b) does not pass through a proximal end-opening 322 of guidewire lumen 316. For some of these applications, a center 323 of distal end-opening 320 of guidewire lumen 316 is disposed within 1 mm of central longitudinal axis 318 of tip 308, e.g., center 323 falls on central longitudinal axis 318. Alternatively or additionally, for some of these applications, central longitudinal axis 318 of tip 308 passes through the distal end of wire 150.

In configurations in which tissue-coupling element 128 comprises tip 308, the tip temporarily serves as an atraumatic distal end of deployment shaft 34 of deployment tool 30 when the tip is removably coupled to a distal end 342 of deployment shaft 34 of deployment tool 30, as shown in Figs. 2A-B. Thus, in these configurations, deployment tool 30 (including deployment shaft 34) is typically not shaped so as define sharp distal piercing tip 32.

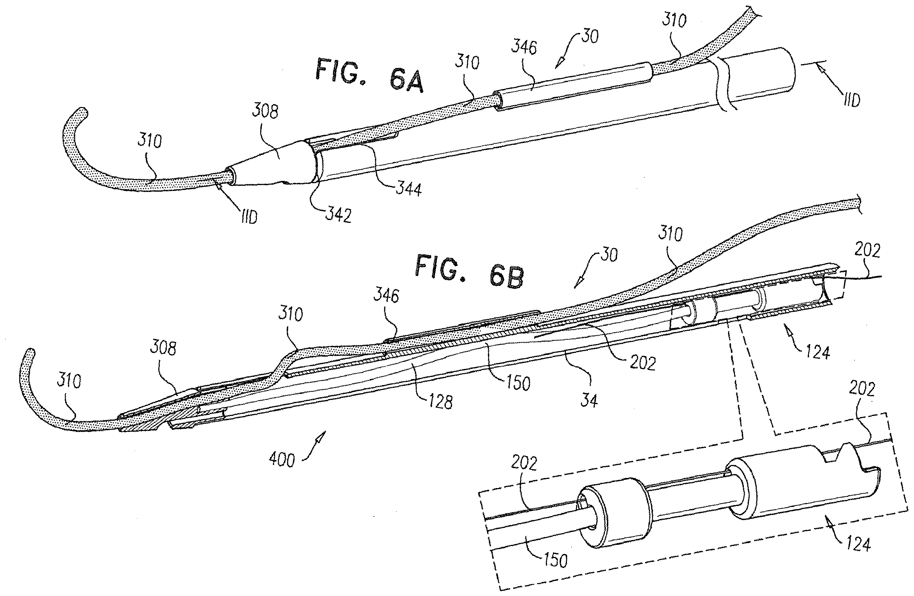

For some applications, deployment shaft 34 of deployment tool 30 has a deployment-shaft outer cross-sectional area which equals between 90% and 110% (e.g., 100%) of the greatest tip outer cross-sectional area, and tip 308 is shaped so as to removably engage distal end 342 of deployment shaft 34, such as shown in Figs. 2A-B, as well as in Figs. 6A-B, described hereinbelow.

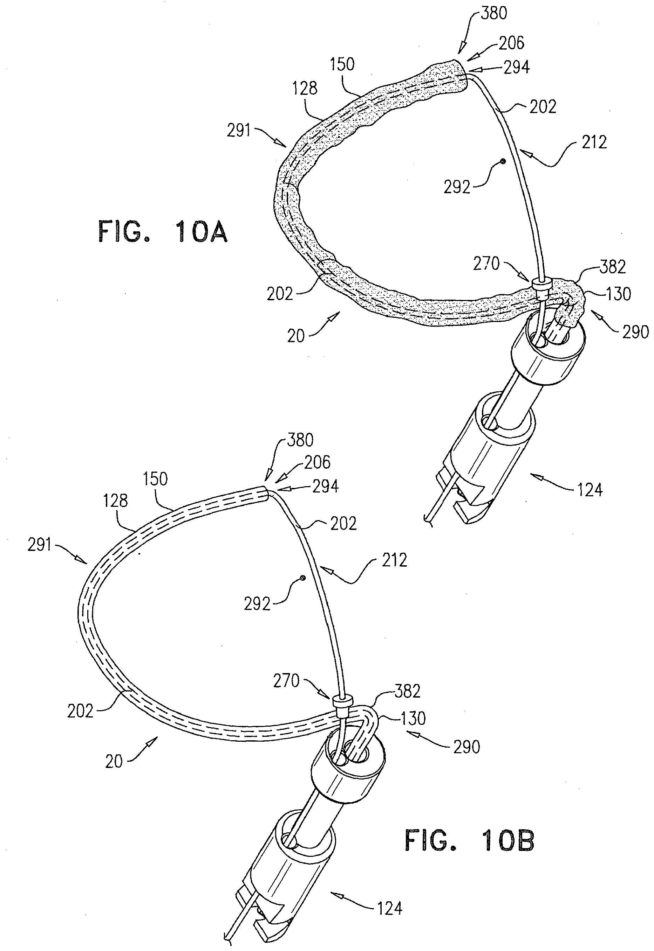

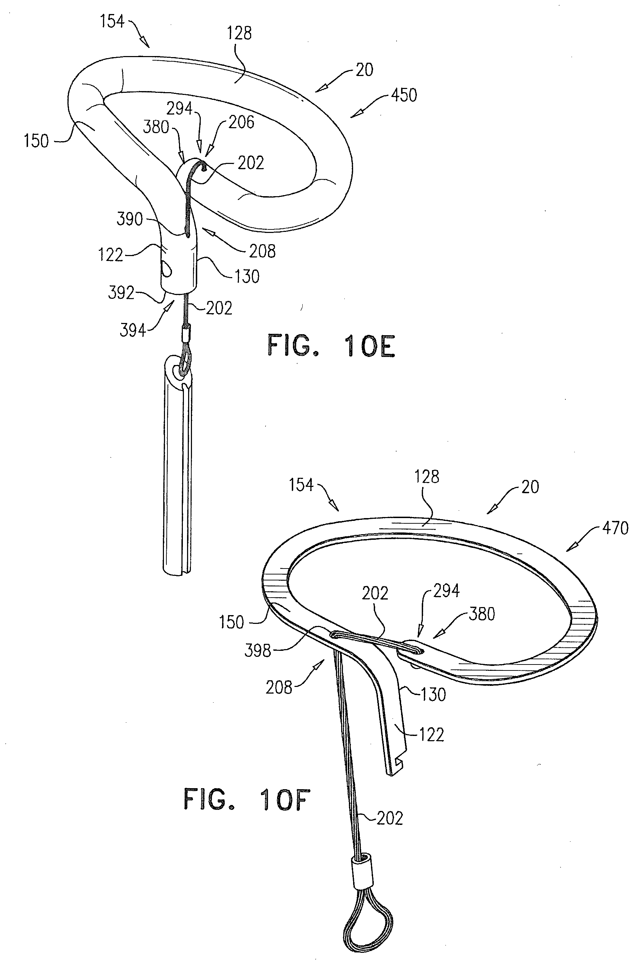

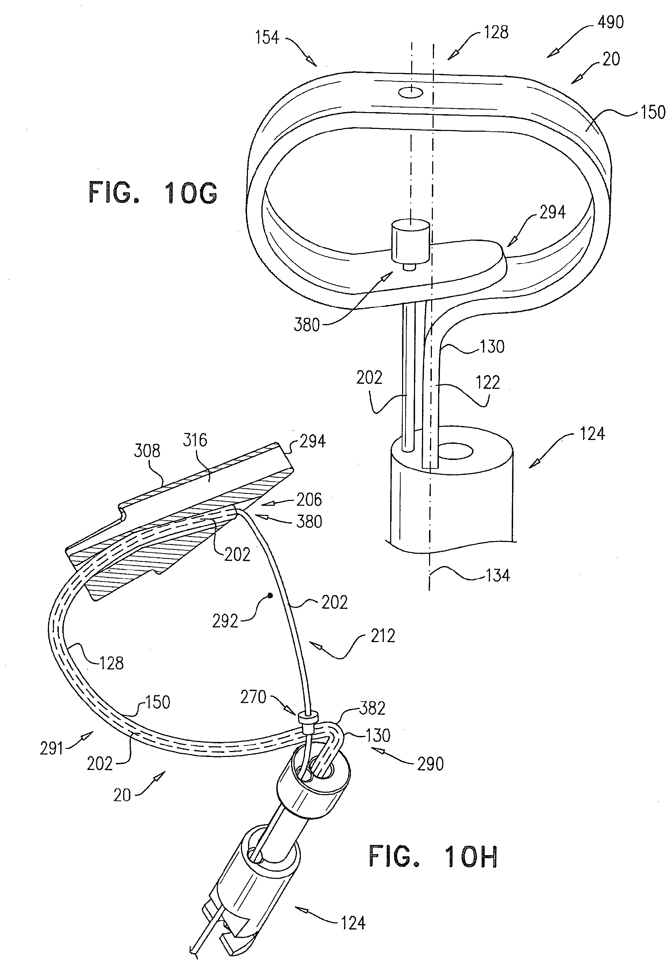

Although only the tissue anchors illustrated in Figs. 2A-3C, 4A-B, 5A-D, 7, 9A-C, 10H, and 15A-C are shown comprising tip 308, the tissue anchors illustrated in Figs. 1A-D, 9D-F, 10A-D, 10F, 12, 13A-E, 14A-D, and 16 may optionally also comprise tip 308 (such as described below with reference to Fig. 10H regarding Figs. 10A-D and 10F).

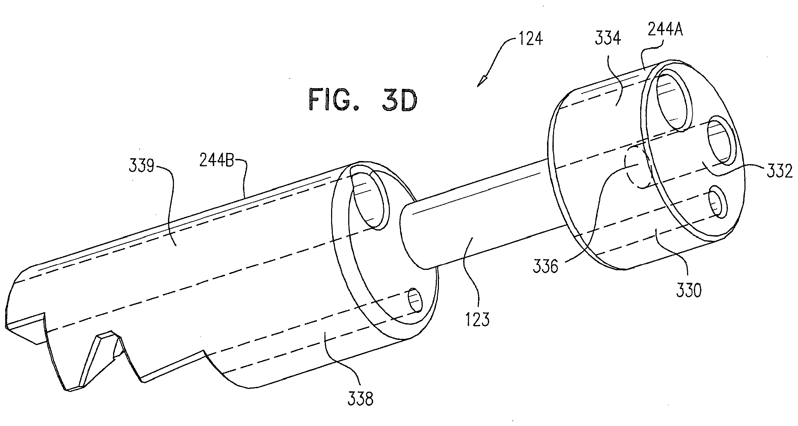

Reference is now made to Fig. 3D, which is a schematic illustration of anchor head 124 of tissue anchor 300.

For clarity of illustration, flexible elongate tension member 202, wire 150 of tissue-coupling element 128, and guidewire 310 are not shown in Fig. 3D; these elements are shown *inter alia* in Fig. 3B. As mentioned above with reference to Figs. 1A-D, for some applications, anchor head 124 comprises distal guide collar 244A and proximal driver collar 244B, which are optionally connected by a collar shaft 123.

For some applications, anchor head 124 (e.g., distal guide collar 244A thereof, as shown) is shaped so as to define:
- a first passage 330, in which proximal portion 208 of flexible elongate tension member 202 is slidably disposed,
- a second passage 332, in which a proximal portion of wire 150 of tissue-coupling element 128 is fixedly disposed, and
- a third passage 334, sized for slidable passage therethrough of guidewire 310.

First, second, and third passages 330, 332, and 334 have respective, different central longitudinal axes. The passages keep flexible elongate tension member 202, the proximal portion of wire 150, and guidewire 310 aligned with but separate from one another. For some applications, a proximal end 336 of second passage 332 is closed. For some applications, third passage 334 has an inner diameter of between 0.25 and 0.75 mm.

For some applications, distal guide collar 244A of anchor head 124 is shaped so as to define first, second, and third passages 330, 332, and 334, and proximal driver collar 244B of anchor head 124 to shaped so as to define:
- a fourth passage 338, (a) in which proximal portion 208 of flexible elongate tension member 202 is slidably disposed, and (b) which is optionally coaxial with first passage 330 (as shown), and/or
- a fifth passage 339, (a) which is sized for slidable passage therethrough of guidewire 310, and (b) which is optionally coaxially with third passage 334 (as shown).

For some applications, when tissue anchor 300 (and open loop 154 (e.g., spiral 160) thereof) is unconstrained by deployment tool 30, open loop 154 (e.g., spiral 160) has a first outer dimension, measured in a direction parallel to flexible elongate tension member 202. After tension is applied to flexible elongate tension member 202, flexible elongate tension member 202 becomes more narrow in the direction of flexible elongate tension member 202, such that open loop 154 (e.g., spiral 160) has a second outer dimension, measured in a direction parallel to flexible elongate tension member 202, which is less than the first outer dimension, e.g., no more than 90% of the first out dimension, such as no more than 80% of the first out dimension, e.g., no more than 70% of the first out dimension, no more than 50% of the first out dimension, or no more than 20% of the first out dimension. For some applications, the force applied to flexible elongate tension member 202 to achieve this reduction is between 2 and 50 N, such as between 5 and 20 N, e.g., 5 N, 7 N, 10 N, 20 N, or 30 N. The amount of force is dependent on the radius of wire 150, and may increase as a power of the radius, such as a third or fourth power of the radius. For some applications, a smallest radius of wire 150 is chosen that is able to withstand between 5 and 20 N of force.

Reference is now made to Figs. 4A-B, which are schematic illustrations of two configurations of a tissue anchor system 248.

In these applications, tissue anchor 300 is a first tissue anchor 182A of tissue anchor system 248, which further comprises (a) a second tissue anchor 182B, which is separate and distinct first tissue anchor 182A, and (b) the one or more tethers 132, which are configured to couple (i) flexible elongate tension member 202 of first tissue anchor 182A to (ii) second tissue anchor 182B. For some applications, one of the one or more tethers 132 is fixed to (a) flexible elongate tension member 202 of first tissue anchor 182A and (b) second tissue anchor 182B.

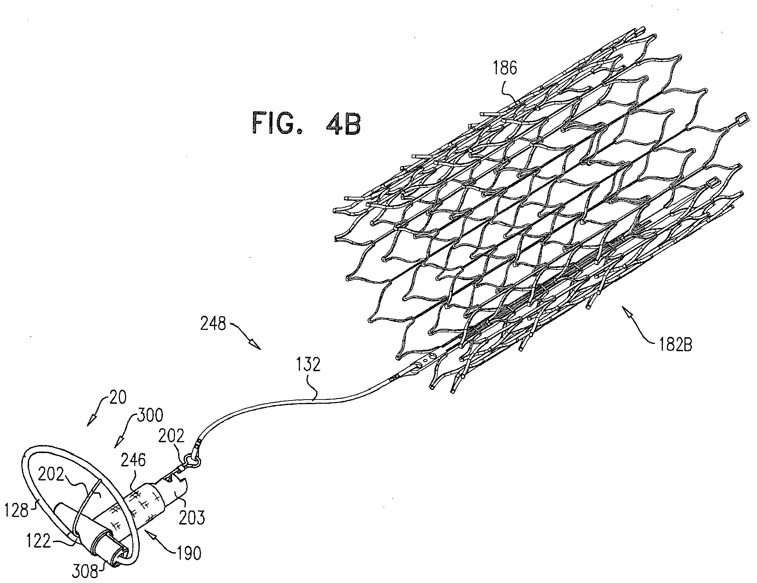

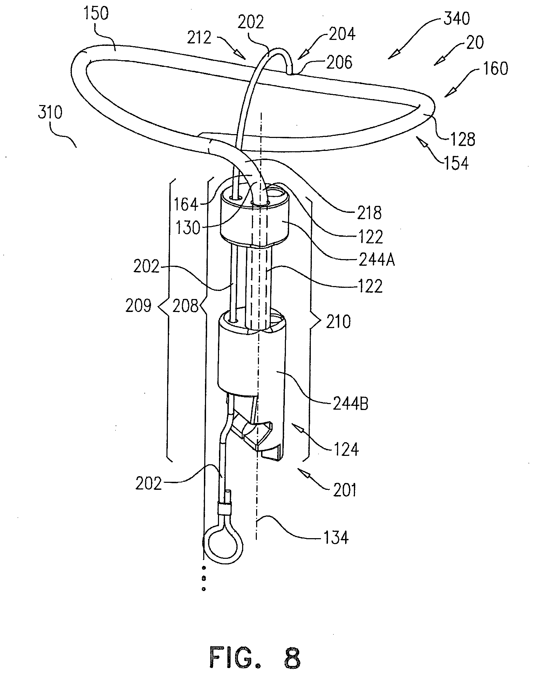

For some applications, such as shown in Fig. 4A, second tissue anchor 182B comprises a helical tissue-coupling element 184. For example, second tissue anchor 182B may implement techniques described in PCT Publication WO 2015/193728 (e.g., with reference to Fig. 8 thereof), PCT Publication WO 2014/108903, and/or the patents and patent application publications referenced below. For other applications, such as shown in Fig. 4B, second tissue anchor 182B comprises a stent 186. For example, second tissue anchor 182B may implement techniques described in one or more of the following applications: US Patent Application Publication 2011/0184510, US Patent Application Publication 2012/0035712, US Patent Application Publication 2013/0018459, US Patent Application Publication 2013/0046380, PCT Publication WO 2014/141239, and/or the patents and patent application publications referenced below.

Reference is made to Figs. 1A-D, 3A, and 4A-B. For some applications, anchor shaft 122 comprises sealing element 190, which is configured to form a blood-tight seal between a portion of anchor shaft 122 inside the heart chamber and wall 194 of the heart. For some applications, sealing element 190 is annular, and snugly surrounds anchor shaft 122.

Reference is now made to Figs. 5A-B, which are schematic illustrations of tissue-coupling element 128 and anchor shaft 122.

Figs. 5A-B provide two views of a configuration tissue-coupling element 128 and anchor shaft 122, when tissue anchor 300 is unconstrained by deployment tool 30. The parameters of open loop 354 of tissue anchor 300 described with reference to Figs. 5A-B may also apply to open loop 154 of tissue anchor 200, described hereinabove with reference to Figs. 1A-D.

Reference is made to Figs. 3A-C and 5A-D. When tissue anchor 300 is unconstrained by deployment tool 30, such as shown in Figs. 3A-C and 5A-D:
- anchor shaft 122 has central longitudinal axis 134,
- anchor head 124 is coaxial with central longitudinal axis 134, and
- tissue-coupling element 128 is shaped such that if tissue-coupling element 128 were to be projected onto a plane 136 that is perpendicular to central longitudinal axis 134:
   ▪ at least 80% (e.g., at least 90%, such as at least 95%) of an area 138 of a projection 139 of tissue-coupling element 128 on plane 136 would fall within a first angle α (alpha) of 180 degrees in plane 136 having a vertex 140 at central longitudinal axis 134, as labeled in Fig. 2B, and
   ▪ area 138 would partially overlap, at a distance D1 of at least 3 mm from vertex 140, both rays 142A and 142B of a second angle β (beta) of between 45 and 180 degrees in plane 136 having vertex 140 at central longitudinal axis 134 (the partial overlap is illustrated by the heavier portions of the rays).

As used in the present application, including in the claims, a "central longitudinal axis" of an elongate structure is the set of all centroids of transverse cross-sectional sections of the structure along the structure. Thus the cross-sectional sections are locally perpendicular to the central longitudinal axis, which runs along the structure. (If the structure is circular in cross-section, the centroids correspond with the centers of the circular cross-sectional sections.)

Tissue-coupling element 128 is configured to have a predetermined shape when unconstrained by deployment tool 30. For example, the tissue-coupling element may comprise a shape-memory material, such as a shape-memory alloy, e.g., Nitinol. Thus, tissue-coupling element 128 automatically transitions to the predetermined shape when released from being constrained by deployment tool 30 to being unconstrained by deployment tool 30.

For some applications, central longitudinal axis 134 is straight when tissue anchor 300 is unconstrained by deployment tool 30, such as shown in Figs. 3A-C and 5A-D.

For some applications, such as shown in Figs. 3A-C and 5A-B, a proximally-facing surface defined by tissue-coupling element 128 (i.e., the surface defined by tissue-coupling element 128 that is configured to touch the external surface of the heart) is concave when tissue anchor 300 is unconstrained by deployment tool 30 (in other words, tissue-coupling element 128 is concave when viewed from proximal portion 126 of anchor shaft 122). Such a concave shape may approximate the natural convex shape of an external surface of the wall of the heart.

For other applications, the proximally-facing surface defined by tissue-coupling element 128 is generally flat, when tissue anchor 300 is unconstrained by deployment tool 30 (configuration not shown). Optionally, upon coming into full contact with the external surface of the heart, the proximally-facing surface defined by the tissue-coupling element may assume a concave shape conforming to the convex shape of the external surface of the heart.

For some applications, when tissue anchor 300 is unconstrained by deployment tool 30:
- a greatest longitudinal dimension D2 of tissue-coupling element 128, measured parallel to central longitudinal axis 134, is between 1 and 6 mm (such as between 2 and 5 mm) (labeled in Fig. 5B), and
- a greatest lateral dimension D3 of tissue-coupling element 128, measured perpendicular to central longitudinal axis 134, is between 4 and 25 mm (such as between 5 and 20 mm) (labeled in Fig. 5A).

Typically, a ratio of the greatest longitudinal dimension D2 and greatest lateral dimension D3 is between 1:2 and 1:18, such as between 1:5 and 1:10, e.g., 1:7 when tissue anchor 300 is unconstrained by deployment tool 30.

For some applications, tissue-coupling element 128 has a length of at least 5 mm (e.g., at least 10 mm), no more than 100 mm (e.g., no more than 60 mm), and/or between 5 and 100 mm (e.g., between 10 and 60 mm) when constrained into a straight configuration.

For some applications, when tissue anchor 300 is unconstrained by deployment tool 30, such as shown in Figs. 3A-C and 5A-D, tissue-coupling element 128 (including wire 150 and tip 308) (a) is shaped as open loop 354 having more than one turn, such that a first complete turn of open loop 354 at least partially overlaps (i.e., runs alongside, above, and/or below) a second at-least-partial turn of open loop 354. For some applications, the first complete turn and the second at-least-partial turn radially coincide, i.e., are at a same distance as each other from a center point (configuration not shown). For other applications, as shown in the figures, an outermost turn of open loop 354 at least partially overlaps (i.e., runs alongside, above, and/or below) a second-to-outermost turn of open loop 354. For some applications, open loop 354 is a three-dimensional open loop. For some applications, open loop 354 is a spiral 360 (e.g., a three-dimensional spiral).

For some applications, open loop 354 extends from distal end 130 of anchor shaft 122 at radially-outer end 164 of open loop 354. For some applications, wire 150 intersects center point 162 (labeled in Fig. 3C) when tissue anchor 300 is unconstrained by deployment tool 30 (configuration not shown), while for other applications, wire 150 does not intersect center point 162 (labeled in Fig. 3C) when tissue anchor 300 is unconstrained by deployment tool 30 (as shown).

For some applications, such as shown in Figs. 3A-C and 5A-D (as well as Figs. 4A-B, 7, 9A-C, and 15A-C), when tissue anchor 300 is unconstrained by deployment tool 30, open loop 354 has more than one turn and less than two turns. For example, as shown in Figs. 3A-C and 5A-D, open loop 354 may have more than one turn and less than 1.5 turns, such as more than one turn, e.g., more than 1.01 turns (363.6 degrees), such as more than 1.02 turns (367.2 degrees), and/or less than 1.25 turns (450 degrees).

For some applications, when tissue anchor 300 is unconstrained by deployment tool 30, such as shown in Figs. 3A-C and 5A-D, tissue-coupling element 128 (including wire 150 and tip 308 if provided) of open loop 354 is shaped as spiral 160 (e.g., a three-dimensional spiral) around center point 162. For some of these applications, wire 150 of spiral 160 extends from distal end 130 of anchor shaft 122 at radially-outer end 164 of spiral 160. For some applications, wire 150 of spiral 160 intersects center point 162 when tissue anchor 300 is unconstrained by deployment tool 30 (configuration not shown), while for other applications, wire 150 of spiral 160 does not intersect center point 162 when tissue anchor 300 is unconstrained by deployment tool 30 (as shown). For some applications, spiral 160 is an elliptical spiral when the tissue anchor is unconstrained by deployment tool 30, such as shown in Figs. 3A-C and 5A-D (as well as in Figs. 4A-B, 7, 9A-C, and 15A-C).

For some applications, such as shown in Figs. 3A-C and 5A-D (as well as Figs. 4A-B, 7, 9A-C, and 15A-C), when the tissue anchor is unconstrained by deployment tool 30, spiral 160 has more than one turn and less than two turns. For example, as shown in Figs. 3A-C and 5A-D, spiral 160 may have more than one turn and less than 1.5 turns, such as more than one turn and less than 1.25 turns.

For some applications, when tissue anchor 300 is unconstrained by deployment tool 30, the open loop (e.g., the spiral) has greatest lateral dimension D3 (labeled in Fig. 5A), measured perpendicular to central longitudinal axis 134, and a distance between (a) radially-outer end 164 of open loop 354 (e.g., spiral 160) and (b) a radially-inner-most point 166 of open loop 354 (e.g., spiral 160), measured perpendicular to central longitudinal axis 134, is equal to at least 30% of the greatest lateral dimension D3. Alternatively or additionally, for some applications, a distance between radially-inner-most point 166 and a closest point thereto on an outermost turn of open loop 354, measured perpendicular to central longitudinal axis 134, is equal to at least 30% of the greatest lateral dimension D3.

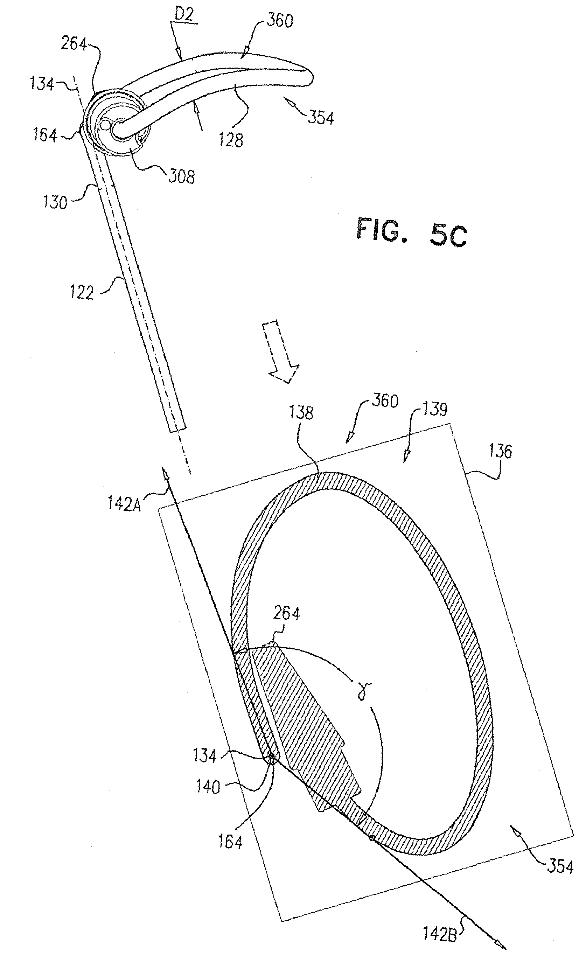

Reference is made to Figs. 5C-D, which are schematic illustrations of tissue-coupling element 128 and anchor shaft 122. The parameters of open loop 354 of tissue anchor 300 described with reference to Figs. 5C-D may also apply to open loop 154 of tissue anchor 200, described hereinabove with reference to Figs. 1A-D. For some applications, as shown in Fig. 5C, at least 80%, such as at least 90%, e.g., at least 95%, of area 138 of projection 139 of tissue-coupling element 128 on plane 136 would fall within a third angle γ (gamma) of 150 degrees in plane 136 having vertex 140 at central longitudinal axis 134, if tissue-coupling element 128 were to be projected onto plane 136.

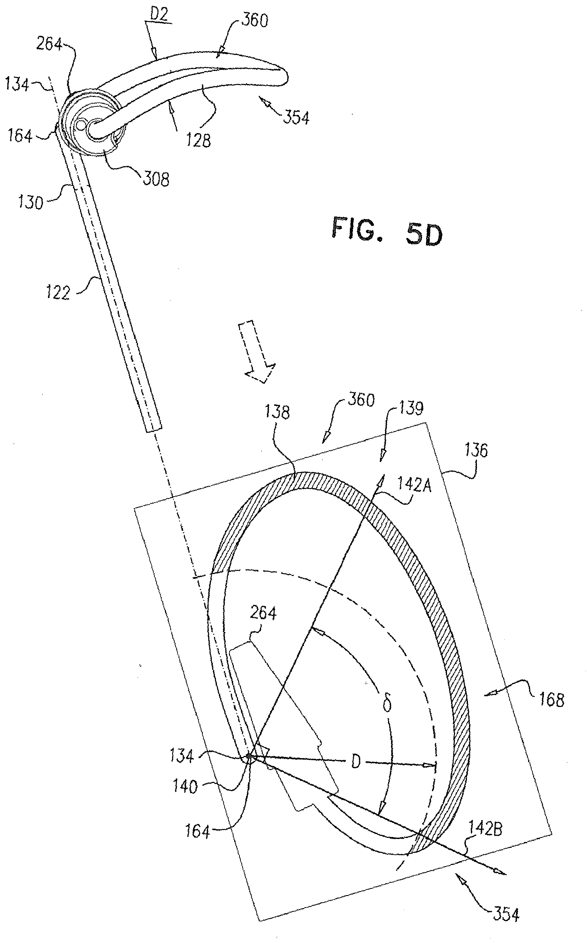

For some applications, as shown in Fig. 5D, an outer portion 168 of area 138 of projection 139 of tissue-coupling element 128 on plane 136 consists of all points of area 138 at least a distance D from vertex 140; for example, the distance D may be 2 mm, such as 3 mm, e.g., 4 mm. Outer portion 168 would fall within all angular positions of a fourth angle δ (delta) of 90 degrees in plane 136 having vertex 140 at central longitudinal axis 134, which outer portion 168, if tissue-coupling element 128 were to be projected onto plane 136. In other words, at all angular positions of fourth angle δ (delta), there is at least one point of outer portion 168. (Outer portion 168 may additionally fall within angular positions outside of fourth angle δ (delta), such as shown in Fig. 5D.)

Reference is now made to Figs. 6A-B, which are schematic illustrations of a tissue anchor 400 before deployment from deployment tool 30.

Tissue anchor 400 is one implementation of tissue anchor 20, described above. Other than as described below and shown in the figures, tissue anchor 400 is generally similar to tissue anchor 300, described hereinabove with reference to Figs. 2A-B, 3A-D, 4A-B, and 5A-D, and may implement any of the features thereof, *mutatis mutandis.*

In the configuration shown in Figs. 6A-B, guidewire 310, rather than passing through deployment shaft 34 of deployment tool 30 alongside wire 150 of tissue-coupling element 128 (as shown in Figs. 2A-B), exits deployment shaft 34 near distal end 342 of deployment shaft 34, typically through a guidewire opening 344 (e.g., slot) that is defined by the wall of deployment shaft 34 and extends to distal end 342 of deployment shaft 34. Optionally, deployment tool 30 further comprises a guide tube 346, which is fixed to an external surface of deployment tool 30; guidewire 310 passes through guide tube 346. In addition, in the configuration shown in Figs. 6A-B, anchor head 124 typically is not shaped so as to define third passage 334 (described hereinabove with reference to Fig. 3D), because guidewire 310 does not pass through anchor head 124.

Reference is now made to Fig. 7, which is a schematic illustration of a tissue anchor 430. Tissue anchor 430 is one implementation of tissue anchor 20, described above. Other than as described below, tissue anchor 430 is generally similar to tissue anchor 300, described hereinabove with reference to Figs. 2A-B, 3A-D, 4A-B, and 5A-D, and may implement any of the features thereof, *mutatis mutandis.* In addition, tissue anchor 430 may implement any of the features of tissue anchor 200, described hereinabove with reference to Figs. 1A-D, *mutatis mutandis,* and/or tissue anchors 340, 350, 430, 370, 400, 420, 470, and/or 490, *mutatis mutandis.*

Tissue-coupling element 128 of tissue anchor 430 comprises wire 150, which is shaped as an open loop 256, e.g., a spiral 260. Wire 150 extends from distal end 130 of anchor shaft 122 at a radially-inner end 264 of open loop 256 (e.g., spiral 260), when tissue anchor 220 is unconstrained by deployment tool 30. This is unlike the typical configurations of open loop 154 (e.g., spiral 160) and open loop 354 (e.g., spiral 360), described hereinabove, in which wire 150 extends from distal end 130 of anchor shaft 122 at radially-outer end 164 of the open loop (e.g., the spiral). In the present configurations, when tissue anchor 430 is unconstrained by deployment tool 30, radially-inner end 264 of open loop 256 (e.g., spiral 260) is typically disposed within 15 mm of center point 162, such as coinciding with center point 162.

Typically, tissue anchor 430 comprises exactly one flexible elongate tension member 202, which includes:
- distal portion 204 that is fixed to site 206 on open loop 256 (e.g., spiral 260),
- longitudinal segment 209 of proximal portion 208 that runs alongside the at least a portion 210 of anchor shaft 122 (labeled, for example, in Fig. 3B), and
- crossing portion 212, which (a) is disposed between distal and proximal portions 204 and 208 (labeled, for example, in Fig. 3B) along flexible elongate tension member 202, and (ii) crosses at least a portion of open loop 256 (e.g., spiral 260) when tissue anchor 430 is unconstrained by deployment tool 30.

For some applications, as shown, site 206 is on outermost turn 214 of open loop 256 (e.g., spiral 260), when tissue anchor 430 is unconstrained by deployment tool 30. Flexible elongate tension member 202 may implement any of the features described hereinabove with reference to Figs. 1A-5D, *mutatis mutandis.*

Reference is now made to Fig. 8, which is a schematic illustration of a tissue anchor 340 after deployment from deployment tool 30.

Tissue anchor 340 is one implementation of tissue anchor 20, described above. Other than as described below and shown in the figures, tissue anchor 340 is generally similar to tissue anchor 300, described hereinabove with reference to Figs. 2A-B, 3A-D, 4A-B, and 5A-D, and may implement any of the features thereof, *mutatis mutandis.* Tissue anchor 340, unlike tissue anchor 300, does not comprise tip 308; therefore, tissue anchor 340 is typically delivered using deployment shaft 34 having sharp distal piercing tip 32, as described hereinabove with reference to Fig. 1A.

Reference is made to Figs. 9A-F, which are schematic illustrations of alternative ways to fix flexible elongate tension member 202 to site 206 of open loop 154 or open loop 354.

These techniques may be used in combination with any of the configurations of tissue anchor 20 described herein.

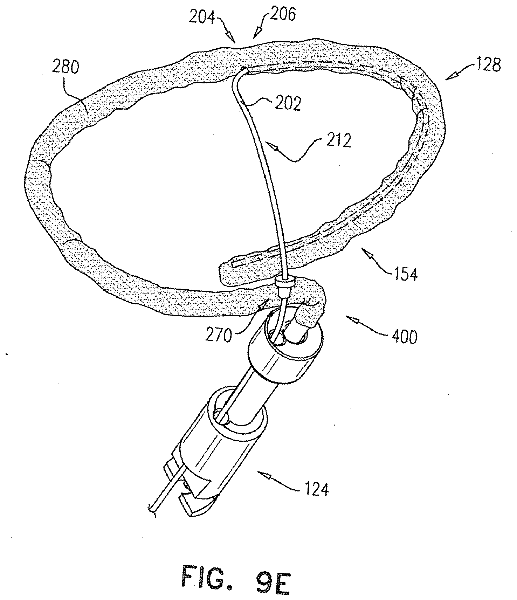

In the configuration shown in Fig. 9A, distal portion 204 of flexible elongate tension member 202 is fixed to site 206 on open loop 354 by crimping a crimping element 288 around wire 150. In this configuration, a distal portion of flexible elongate tension member 202 beyond site 206 is fixed (e.g., by welding or soldering) to open loop 354, such as near radially-inner end 264 of open loop 354. Distal portion 204 of flexible elongate tension member 202 at least partially runs along open loop 354 between site 206 (on open loop 354) and tip 308. The portion of flexible elongate tension member 202 between site 206 and radially-inner end 264 may be attached to wire 150, or may be held near wire 150, such as by a sleeve, as described with reference to Fig. 9E. It is noted that site 206 is the site on open loop 354 at which flexible elongate tension member 202 makes functional contact with the loop for applying tension across the loop, rather than other sites along wire 150 to which flexible elongate tension member 202 may also be attached.

Although the techniques of Fig. 9A are illustrated for open loop 354 of tissue anchor 300, these techniques may also be practiced with the other tissue anchors described herein, including with open loop 154 of tissue anchor 200, described hereinabove with reference to Figs. 1A-D.

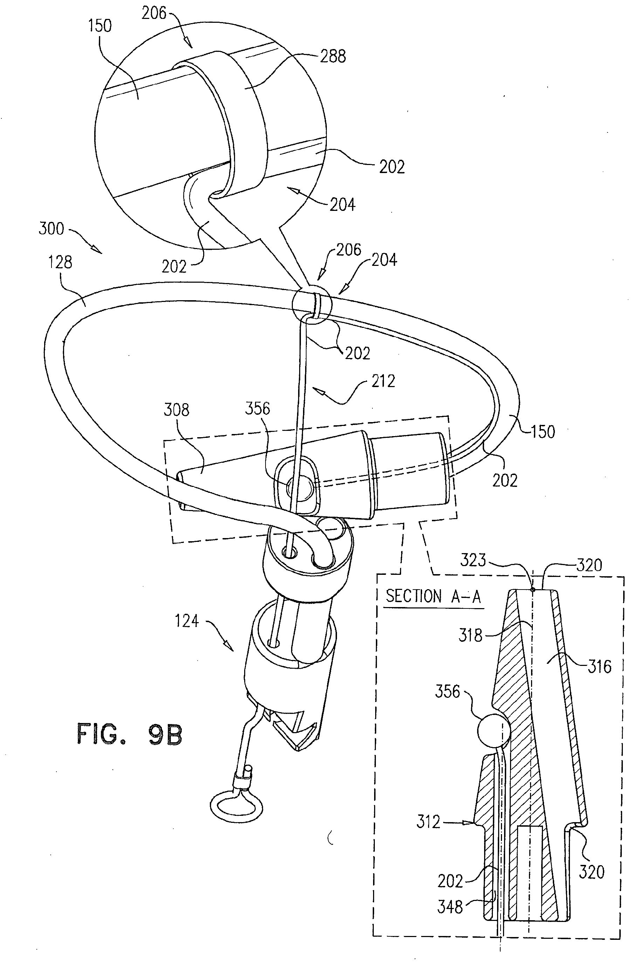

The configuration shown in Fig. 9B is generally similar to the configuration shown in Fig. 9A, except that in the configuration shown in Fig. 9B, distal portion 204 of elongate tension member 202, at one or more locations along distal portion 204, is fixed to tip 308. For some applications, tip 308 is shaped so as to define a tension-member lumen 348 therethrough, and distal portion 204 of flexible elongate tension member 202 passes through at least a portion of tension-member lumen 348. For some applications, flexible elongate tension member 202 is fixed to tip 308 by a bead 356 soldered to a distal end of flexible elongate tension member 202.

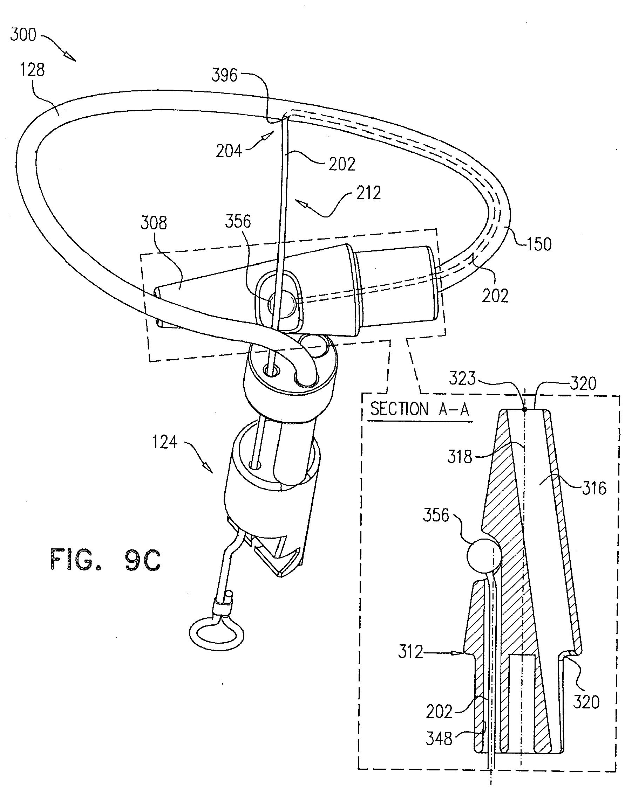

The configuration shown in Fig. 9C is generally similar to the configuration shown in Fig. 9B, except that in the configuration shown in Fig. 9C, wire 150 is shaped so as to define a channel (i.e., wire 150 is tubular), and the channel is shaped so as to define a lateral opening 396 at site 206. Distal portion 204 of flexible elongate tension member 202 enters the channel through lateral opening 396 at site 206, passes through the channel, and exits wire 150 at distal end 294 of wire 150.

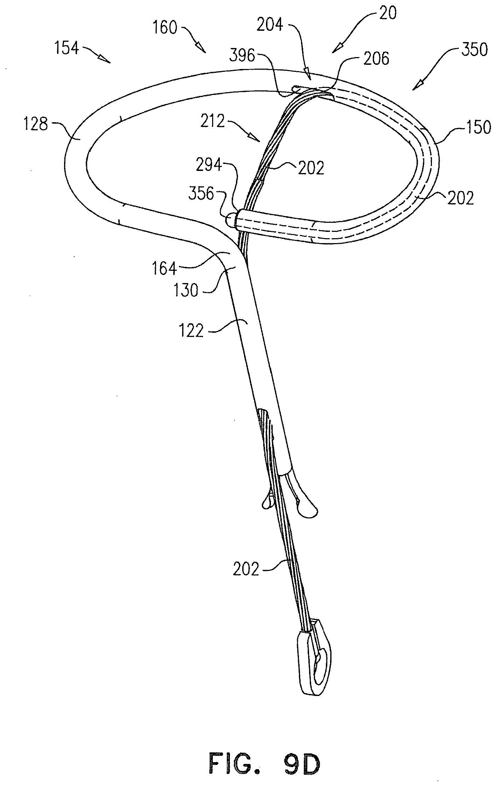

Fig. 9D is a schematic illustration of a tissue anchor 350.

Tissue anchor 350 is one implementation of tissue anchor 20, described above. Tissue anchor 350 is generally similar to the configuration of tissue anchor 300 shown in Fig. 9C, except as follows. Like the configuration shown in Fig. 9C, wire 150 is shaped so as to define a channel which has lateral opening 396 at site 206. Distal portion 204 of flexible elongate tension member 202 enters the channel through lateral opening 396 at site 206, passes through the channel, and exits wire 150 at distal end 294 of wire 150. For some applications, flexible elongate tension member 202 is fixed to distal end 294 of wire by bead 356 soldered to a distal end of flexible elongate tension member 202.

The configuration shown in Fig. 9E may be used in combination with the configuration shown in Fig. 9A, 9B, 9C, or 9D, or separately. In the configuration shown in Fig. 9E, open loop 154 is covered with a sleeve 280, which may comprise a woven material, comprising, for example, polyester. A distal portion of flexible elongate tension member 202 beyond site 206 is fixed (e.g., by welding or soldering) to open loop 154, such as near radially-inner end 264 of open loop 154 (this area of open loop 154 may facilitate attachment because this area is straighter than other portions of the open loop). Flexible elongate tension member 202 penetrates and exits sleeve 280 at site 206, such as by passing between the fibers of sleeve 280, or through an opening made in sleeve 280, which opening is optionally reinforced. Distal portion 204 of flexible elongate tension member 202 is fixed to site 206 on open loop 154 indirectly by being restrained by sleeve 280. Sleeve 280 may in addition improve tissue growth on the tissue anchor. Optionally, a more proximal portion of flexible elongate tension member 202, after crossing open loop 154, re-enters sleeve 280 through a lateral wall of the sleeve, and exits the proximal end of the sleeve.

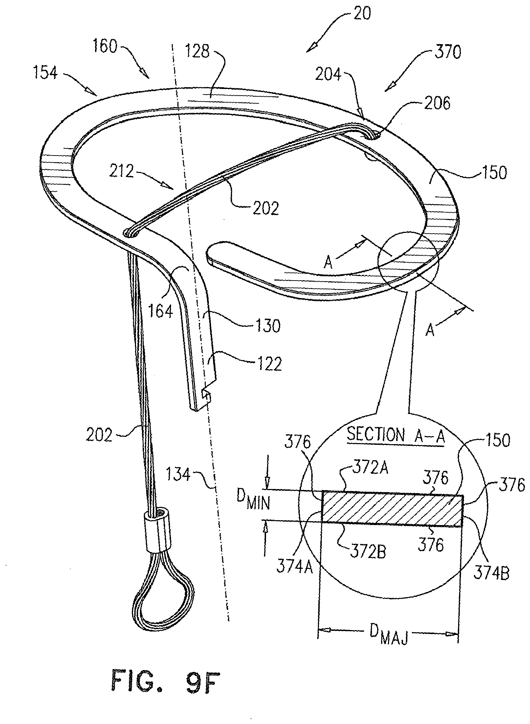

Fig. 9F is a schematic illustration of a tissue anchor 370.

Tissue anchor 370 is one implementation of tissue anchor 20, described above. Tissue anchor 370 is similar in some respect to tissue anchor 340, described hereinabove with reference to Fig. 8, and may implement any features thereof, mutatis mutandis. Tissue anchor 370 optionally does not comprise anchor head 124, as shown; alternatively, tissue anchor 370 does comprise anchor head.

For some applications, wire 150 of tissue anchor 370 is shaped so as to define first and second major opposing surfaces 372A and 372B connected by first and second minor opposing surfaces 374A and 374B. First and second major opposing surfaces 372A and 372B and first and second minor opposing surfaces 374A and 374B extend along at least 90% of a total length of wire 150. A total surface area of first minor opposing surface 374A is less than 20%, e.g., less than 10%, such as less than 5%, of a total surface area of major opposing surface 372A.

Alternatively or additionally, for some applications, extending along at least 90% of a total length of wire 150, wire 150 has a greatest major dimension D_{MAJ} and a greatest minor dimension D_{MIN} perpendicular to the greatest major dimension D_{MAJ}. The greatest major dimension D_{MAJ} equals at least 150% (e.g., at least 200%, such as at least 300%) of the greatest minor dimension D_{MIN}.

Alternatively or additionally, for some applications, at a plurality of locations along wire 150, a cross section of wire 150, taken perpendicular to a longitudinal axis of wire 150, has a shape that has at least one straight side 376, such as at least two straight sides 376, at least three straight sides 376, or four straight sides 376 (as shown). For some applications, the at least one straight side 376 has a length of at least 3 mm. (It is noted that the longitudinal axis of wire 150 typically includes one or more curved portions, such as shown in Fig. 9F.)

Reference is still made to Fig. 9F. For some applications, when the tissue anchor 370 is unconstrained by deployment tool 30: (a) a greatest longitudinal dimension of an open shape 291 (e.g., open loop 154), measured in parallel to a central longitudinal axis of anchor shaft 122, is between 0.25 and 5 mm, e.g., between 1 and 5 mm, and (b) a greatest lateral dimension of open shape 291 (e.g., open loop 154), measured perpendicular to the central longitudinal axis, is between 4 and 20 mm.

Reference is now made to Figs. 10A-H, which are schematic illustrations of respective configurations of tissue anchor 20.

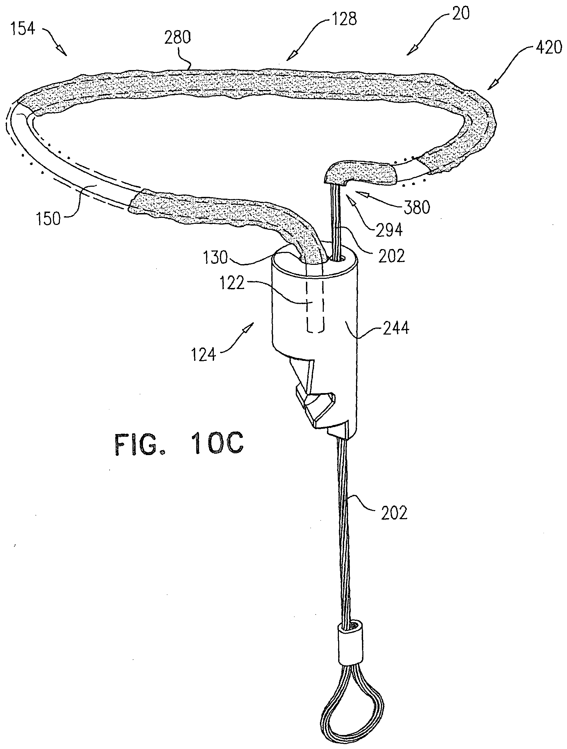

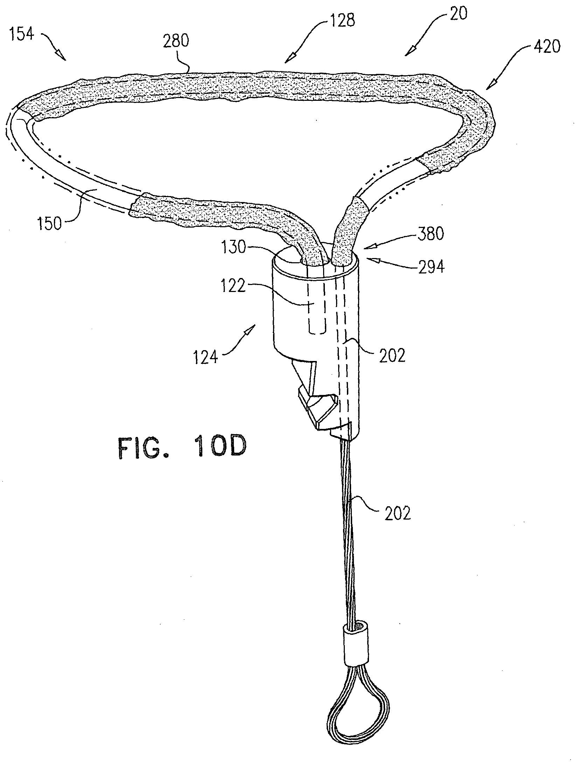

Tissue anchor 20 may have any of the characteristics described herein, *mutatis mutandis.* In these configurations of tissue anchor 20, tissue-coupling element 128 (e.g., wire 150 thereof, as shown in Figs. 10A-G, or wire 150 and tip 308 thereof together, as shown in Fig. 10H) is shaped as open shape 291, such as open loop 154, when tissue anchor 20 is unconstrained by deployment tool 30. Flexible elongate tension member 202 extends from a distal site 380 on open shape 291 (in other words, site 206 is distal site 380), distal site 380 located within 7 mm (e.g., within 3 mm) of distal end 294 of open shape 291, such as at distal end 294, as shown in Figs. 10A, 10B, 10C, and 10D). Alternatively or additionally, distal site 380 is located within a distance of distal end 294 of open shape 291, which distance equals 5% of a total length of wire 150, and/or equals 10% of greatest lateral dimension D3 of tissue-coupling element 128, measured perpendicular to central longitudinal axis 134 (labeled *inter alia* in Fig. 5A).

As described hereinabove, proximal portion 208 of flexible elongate tension member 202 has longitudinal segment 209 that runs alongside at least portion 210 of anchor shaft 122 when tissue anchor 20 is unconstrained by deployment tool 30 (these elements are labeled in Figs. 1C and 3B, described hereinabove). As described hereinabove, tissue anchor 20 is configured to allow relative axial motion between the at least a portion 210 of anchor shaft 122 and longitudinal segment 209 of proximal portion 208 of flexible elongate tension member 202 when tissue anchor 20 is unconstrained by deployment tool 30.

For some applications, application to flexible elongate tension member 202 of a distally-directed force of at least 1 N while tissue anchor 20 is unconstrained draws the distal end of open shape 291 toward distal end 130 of anchor shaft 122.

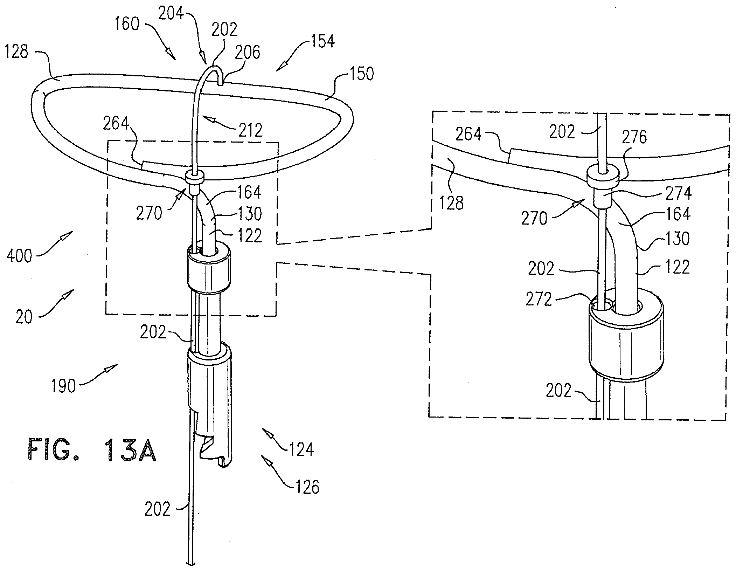

Fig. 10A is a schematic illustration of a tissue anchor 290, in accordance with an application of the present invention. Tissue anchor 290 is one implementation of tissue anchor 20, described above. Except as described below, tissue anchor 290 is generally similar to tissue anchor 340, described hereinabove with reference to Fig. 8, and tissue anchor 400, described hereinbelow with reference to Figs. 13A-E. Wire 150 of tissue anchor 290 is not shaped as open loop 154 when tissue anchor 290 is unconstrained by deployment tool 30. Instead, wire 150 is shaped as open shape 291, such as a portion of a circle or a portion of an ellipse. Typically, if, when tissue anchor 290 is unconstrained by deployment tool 30, tissue-coupling element 128 were to be projected onto plane 136 that is perpendicular to central longitudinal axis 134 of anchor shaft 122, open shape 291 would surround at least 170 degrees, no more than 355 degrees, and/or between 170 and 355 degrees of a point 292 in plane 136, such as at least 180 degrees (e.g., at least 190 degrees), no more than 345 degrees, and/or between 180 degrees (e.g., 190 degrees) and 345 degrees. For some applications, such as in which open shape 291 surrounds between 170 and 190 degrees of point 292, site 206 is at distal end 294 of wire 150. For some of these applications, wire 150 is shaped so as to define a channel (i.e., wire 150 is tubular). A portion of flexible elongate tension member 202 passes through the channel and exits the channel of wire 150 at distal end 294 of wire 150. For any of the applications described above, point 292 optionally may fall on a projection onto plane 136 of a line segment that terminates at (a) site 206 on wire 150 and (b) a proximal end 382 of tissue-coupling element 128 when tissue anchor 290 is unconstrained by deployment tool 30. For any of the applications described above, when tissue anchor 290 is unconstrained by deployment tool 30, (a) a line segment that terminates at (i) site 206 (i.e., distal site 380) on open shape 291 and (ii) proximal end 382 of tissue-coupling element 128 may have a total length that equals a percentage of (b) a total length of tissue-coupling element 128, measured along tissue-coupling element 128, the percentage at least 25%, (e.g., at least 40%, such as at least 50%), no more than 75% (e.g., no more than 70%, such as no more than 60%), and/or between 25% and 75% (e.g., between 40% and 70%, such as between 50% and 60%). Typically, at least a portion of tissue-coupling element 128 is curved, such as the entire tissue-coupling element 128.

For some applications, such as shown in Figs. 10A-E and 10H, wire 150 is shaped so as to define a channel (i.e., wire 150 is tubular). In these applications, flexible elongate tension member 202 typically passes through at least a portion of the channel. For some applications, such as shown in Figs. 10A-D and 10H, flexible elongate tension member 202 passes through the entire channel, and distal site 380 on open shape 291 is a distal- end opening of open shape 291. For some applications, a distal end portion of flexible elongate tension member 202 is fixed at or beyond proximal end 382 of open shape 291 (e.g., fixed to anchor head 124 beyond proximal end 382 of open shape 291).

For some applications, such as shown in Fig. 10B, when tissue anchor 20 is unconstrained by deployment tool 30, (a) a line segment that terminates at (i) site 206 on wire 150 and (ii) a proximal end 382 of tissue-coupling element 128 may have a total length that equals a percentage of (b) a total length of tissue-coupling element 128, measured along tissue-coupling element 128, the percentage at least 25%, (e.g., at least 40%, such as at least 50%), no more than 75% (e.g., no more than 70%, such as no more than 60%), and/or between 25% and 75% (e.g., between 40% and 70%, such as between 50% and 60%). Typically, at least a portion of tissue-coupling element 128 is curved, such as the entire tissue-coupling element 128.

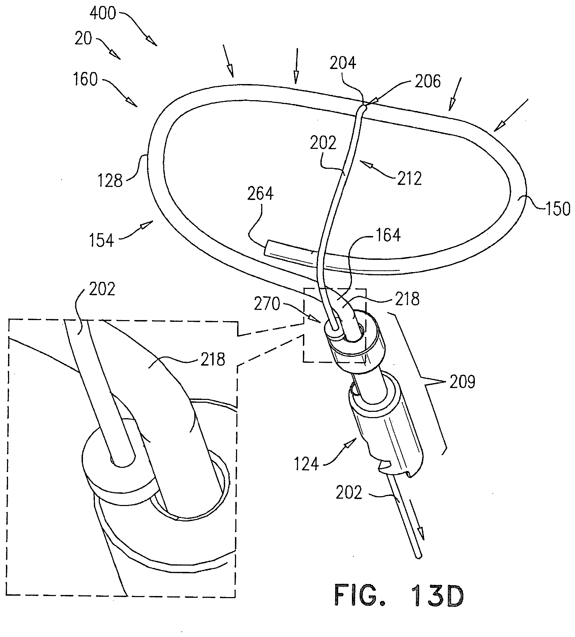

For some of these applications, as shown in Figs. 10C-D, a tissue anchor 420 is provided, which is one implementation of tissue anchor 20. Upon application of distally-directed tension to flexible elongate tension member 202, distal end 294 of wire 150 is drawn into contact with anchor head 124, such as with collar 244 of the anchor head, which prevents excessive, unnecessary strain on open loop 154. This arrangement is similar to, and may serve as an alternative to, locking stopper 270, described hereinbelow with reference to Figs. 13D-E.

For some of these applications, such as shown in Fig. 10E, the channel has a proximal lateral opening 390 within 7 mm of a proximal end 392 of wire 150, and proximal portion 208 of flexible elongate tension member 202 passes through proximal lateral opening 390. For some applications, proximal portion 208 of flexible elongate tension member 202 passes through proximal lateral opening 390 and through a proximal- end opening 394 of the channel.

For some applications, such as shown in Fig. 10E, the channel has lateral opening 396 at site 206, and distal portion 204 of flexible elongate tension member 202 passes through lateral opening 396.

For some applications, such as shown in Fig. 10F, a tissue anchor 470 is provided, which is one implementation of tissue anchor 20. Wire 150 typically has one or more of the shapes and/or dimensions described hereinabove with reference to Fig. 9F.

For some applications, such as shown in Fig. 10F, wire 150 is shaped so as to define a proximal opening 398 within 7 mm (e.g., within 3 mm) of proximal end 392 of wire 150, and proximal portion 208 of flexible elongate tension member 202 passes through proximal opening 398.

For some applications, as shown in Fig. 10G, a tissue anchor 490 is provided, which is one implementation of tissue anchor 20. Open loop 154 generally extends distally from anchor shaft 122. For some applications, wire 150 has one or more of the shapes and/or dimensions described hereinabove with reference to Fig. 9F, except that, unlike the dimensions given for Fig. 9F, typically, when tissue anchor 490 is unconstrained by deployment tool 30: (a) a greatest longitudinal dimension of open shape 291 (e.g., open loop 154), measured in parallel to central longitudinal axis 134 of anchor shaft 122, is between 5 and 15 mm, and (b) a greatest lateral dimension of open shape 291 (e.g., open loop 154), measured perpendicular to central longitudinal axis 134, is between 4 and 20 mm.

Reference is made to Fig. 10H (for clarity of illustration only tip 308 is shown in cross-section). For some applications, the tissue anchors illustrated in Figs. 10A-D and 10F comprise tip 308. For example, Fig. 10H shows a configuration of tissue anchor 290 of Fig. 10B in which the tissue anchor comprises tip 308. For some applications, distal site 380 is on tip 308. For some applications, in this configuration tip 308 is shaped so as define an extension of the channel (the channel is defined by wire 150). Flexible elongate tension member 202 passes through a portion of tip 308 in this extension of the wire channel, and exits tip 308 (and tissue-coupling element 128) at distal site 380.

Reference is now made to Fig. 11A, which is a schematic illustration of tissue anchor 450, described hereinabove with reference to Fig. 10E, before deployment from deployment tool 30. In this configuration, deployment tool 30 does not comprise a lumen in which the tissue anchor is constrained during deployment, but instead comprises a rod 494 that is inserted into the channel of tissue anchor 450 and holds the tissue anchor generally straight for delivery. For some applications, rod 494 is shaped so as to define a guidewire lumen 496, for delivery of deployment tool 30 over a guidewire.

Reference is now made to Fig. 11B, which is a schematic illustration of tissue anchor 470, described hereinabove with reference to Fig. 10F, before deployment from deployment tool 30. The flatness of tissue anchor 470 may leave space in deployment shaft 34 for passage of a guidewire alongside tissue anchor 470.

Reference is now made to Fig. 12, which is a schematic illustration of another configuration of open loop 154. This configuration may be used in combination tissue anchors 20, 200, 300, 340, 350, 430, 370, 400, 420, 470, and 490. In this configuration, when the tissue anchor is unconstrained by deployment tool 30, open loop 154 is shaped so as to define one or more curved portions 296 (e.g., two or more curved portions 296) and one or more straight portions 298 (e.g., two or more straight portions 298). Straight portions 298 generally maximize the surface contact with the external surface of the heart and thus provide good anchoring. For some applications, open loop 154 is shaped as a common, conventional paper clip (an oblong shape with straight sides, with approximately 1.5 turns).

Reference is now made to Figs. 13A-D, which are schematic illustrations of a tissue anchor 400. Tissue anchor 400 is one implementation of tissue anchor 20, described above. Other than as described below, tissue anchor 400 is generally similar to tissue anchor 200, described hereinabove with reference to Figs. 1A-D, and may implement any of the features thereof, *mutatis mutandis.* Alternatively or additionally, tissue anchor 400 may implement any of the features of tissue anchor 300, described hereinabove with reference to Figs. 2A-3D and/or any of the other configurations of tissue anchor 20 described herein, including tissue anchors 200, 340, 350, 430, 370, 420, 470, and 490.

When tissue anchor 400 is unconstrained by deployment tool 30, such as shown in Figs. 13A-D, wire 150 is shaped as open loop 154 (e.g., a three-dimensional open loop) around center point 162 (labeled in Figs. 1D and 3C), and, optionally, as spiral 160 (e.g., a three-dimensional spiral) around center point 162 (labeled in Figs. 1D and 3C). For some applications, such as shown in Figs. 13A-D, wire 150 extends from distal end 130 of anchor shaft 122 at radially-outer end 164 of open loop 154 (and, optionally, spiral 160) (labeled in Fig. 13A), when tissue anchor 400 is unconstrained by deployment tool 30. For some applications, open loop 154 (and, optionally, spiral 160) has the dimensions described hereinabove with reference to Figs. 5A-B and/or 5C-D. For some applications, tissue-coupling element 128 has one or more of the characteristics described hereinabove with reference to Figs. 5C-D. For some applications, the proximally-facing surface defined by tissue-coupling element 128 is generally flat, when tissue anchor 400 is unconstrained by deployment tool 30 (configuration not shown). Optionally, upon coming into full contact with the external surface of the heart, the proximally-facing surface defined by the tissue-coupling element may assume a concave shape conforming to the convex shape of the external surface of the heart.

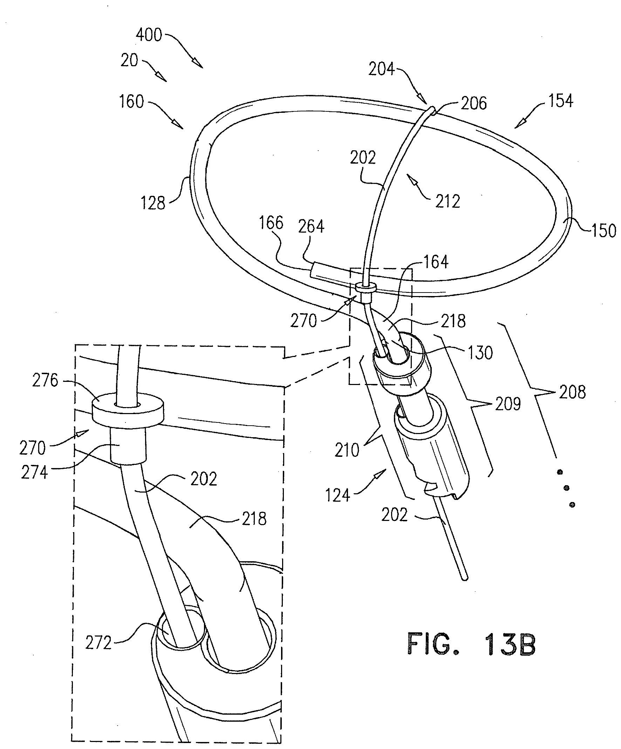

In the configuration shown in Figs. 13A-D, tissue anchor 400 further comprises flexible elongate tension member 202, which includes:
- distal portion 204 that is fixed to site 206 on open loop 154 (such as by welding, soldering, crimping, and/or knotting, and/or as described hereinabove with reference to Figs. 9A-F),
- proximal portion 208, which has longitudinal segment 209 that runs alongside at least portion 210 of anchor shaft 122 (labeled in Fig. 13B, in which the at least a portion 210 of anchor shaft 122 is the entire length of anchor shaft 122), and
- crossing portion 212, which (a) is disposed between distal and proximal portions 204 and 208 along flexible elongate tension member 202, and (ii) crosses at least a portion of open loop 154 when tissue anchor 400 is unconstrained by deployment tool 30.

Although flexible elongate tension member 202 is fixed to wire 150 of tissue-coupling element 128, flexible elongate tension member 202 is typically distinct from wire 150. In other words, flexible elongate tension member 202 and wire 150 are not two longitudinal portions of a single continuous wire, i.e., are not longitudinally contiguous with each other.

Tension is applied to tissue-coupling element 128 of tissue anchor 400 via flexible elongate tension member 202. The applied tension is resisted by the outward force of open loop 154. The applied tension at least partially compresses and stiffens open loop 154. This arrangement of tension distribution may overcome any natural tendency of open loop 154 to straighten (i.e., unwind) if tension were to be applied along central longitudinal axis 134 via anchor shaft 122, and thus may allow the application of a greater load to open loop 154.

Typically, before tension is applied to flexible elongate tension member 202, when tissue anchor 400 is unconstrained by deployment tool 30, flexible elongate tension member 202 is not taut across the at least a portion of open loop 154. For example, flexible elongate tension member 202 may arc distally, such as can best be seen in Fig. 13A.

Typically, tissue anchor 400 is configured to allow relative axial motion between the at least a portion 210 of anchor shaft 122 and longitudinal segment 209 of proximal portion 208 of flexible elongate tension member 202 when tissue anchor 400 is unconstrained by deployment tool 30. Such axial motion allows tension to be applied to flexible elongate tension member 202 without also being applied to anchor shaft 122, and allows open loop 154 to be unwound and flexible elongate tension member 202 to be disposed alongside a portion of flexible elongate tension member 202, as shown in Fig. 13A. Typically, longitudinal segment 209 of proximal portion 208 of flexible elongate tension member 202 is coupled in sliding communication with the at least a portion 210 of anchor shaft 122, when tissue anchor 400 is unconstrained by deployment tool 30. For some applications, tissue anchor 400 comprises one or more annular elements, which are disposed around the at least a portion of anchor shaft 122, and couple flexible elongate tension member 202 in the sliding communication with the at least a portion 210 of anchor shaft 122, when tissue anchor 400 is unconstrained by deployment tool 30. For example, the annular elements may comprise one or more collars, loops, or rings. Anchor shaft 122 (e.g., the collars) is shaped such that flexible elongate tension member 202 runs generally parallel to central longitudinal axis 134 of anchor shaft 122.

For some applications, as shown, site 206 is on an outermost turn of open loop 154, when tissue anchor 400 is unconstrained by deployment tool 30. For some other applications, site 206 is on a second-to-outermost turn of open loop 154, when tissue anchor 400 is unconstrained by deployment tool 30 (configuration not shown).

Typically, a radius of flexible elongate tension member 202 is less than a radius of wire 150, such as less than 50% of the radius of wire 150. Flexible elongate tension member 202 and/or wire 150 may have any of the characteristics described hereinabove with reference to Figs. 3C, 5A-D and/or 5C-D, including dimensions and relative arrangement with respect to each other.

For some applications, one or more tethers 132 are provided, which are configured to be coupled to tissue anchor 400. Typically, the one or more tethers 132 are fixed to flexible elongate tension member 202, typically to proximal portion 208 of the tension member, such as at or near (e.g., within 1 cm of) a proximal end of proximal portion 208. When tension is applied to the one or more tethers, the tension is transmitted to flexible elongate tension member 202, rather than to anchor shaft 122 via anchor head 124.

For some applications, anchor head 124 is shaped so as to define a passage 272 in which proximal portion 208 of flexible elongate tension member 202 is slidably disposed. Flexible elongate tension member 202 comprises a locking stopper 270, which is axially fixed to proximal portion 208 or crossing portion 212 of flexible elongate tension member 202. Locking stopper 270 and passage 272 are sized and shaped such that the size and shape of passage 272 prevent proximal movement of locking stopper 270 past passage 272. Optionally, locking stopper 270 engages passage 272 (as shown). For some applications, passage 272 is a channel through a portion of anchor head 124 (such as through one or more collars of anchor head 124) (as shown), while for other applications, passage 272 is a groove (e.g., a U-shaped groove) (configuration not shown). For some applications, locking stopper 270 is shaped so as to define a base 274 and a flange 276. The flange is too large to pass through passage 272, while base 274 may or may not be too large to enter the passage. For some applications, locking stopper 270 is manufactured as a separate element that is fixed to flexible elongate tension member 202, such as by crimping, welding, or soldering. For other applications, locking stopper 270 is integral to flexible elongate tension member 202.

For some applications, passage 272 extends to a distal end of anchor head 124 (as shown), while for other applications, passage 272 is disposed more proximally in anchor head 124, such as near a proximal end of anchor head 124 (configuration not shown). Typically, locking stopper 270 is axially fixed to proximal portion 208 or crossing portion 212 of flexible elongate tension member 202 at a distance of at least 7 mm, no more than 22 mm, and/or between 7 and 22 mm from site 206 on the open loop, measured along flexible elongate tension member 202 (i.e., measured along the curvature of flexible elongate tension member 202 if it is curved, such as shown in Figs. 13A-B). Alternatively or additionally, for some applications, if tissue-coupling element 128 were straightened in an elongated configuration, for example by being disposed in deployment tool 30 such as shown in Fig. 1A *mutatis mutandis,* locking stopper 270 would be a distance of at least 7 mm, no more than 12 mm, and/or between 7 and 12 mm (e.g., 10 mm) from passage 272. Alternatively or additionally, for some applications, when tissue anchor 400 is unconstrained by deployment tool 30 (and flexible elongate tension member 202 is curved, such as shown in Figs. 13A-B), locking stopper 270 is disposed at a distance of at least 7 mm, no more than 12 mm, and/or between 7 and 12 mm (e.g., 10 mm) from passage 272. For some applications, when sufficient tension is applied to flexible elongate tension member 202 straighten flexible elongate tension member 202 but not compress open loop 154, locking stopper 270 moves between 5 and 8 mm toward passage 272, such that locking stopper 270 is disposed at a distance of at least 2 mm, no more than 5 mm, and/or between 2 and 5 mm (e.g., 10 mm) from passage 272.

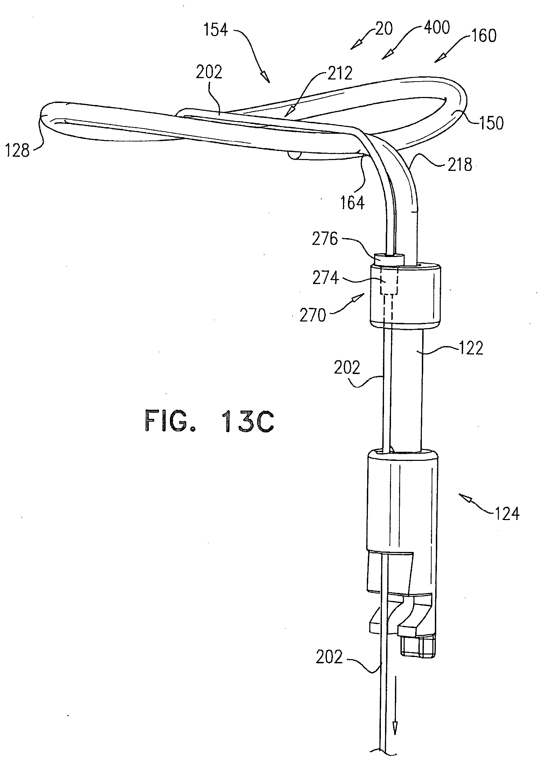

As shown in Fig. 13C-D, tension is applied to tissue-coupling element 128 of tissue anchor 200 via flexible elongate tension member 202. The applied tension is resisted by the outward force of open loop 154. The applied tension at least partially compresses and stiffens open loop 154. This arrangement of tension distribution may overcome any natural tendency of open loop 154 to straighten (i.e., unwind) if tension were to be applied along central longitudinal axis 134 via anchor shaft 122, and thus may allow the application of a greater load to open loop 154. The tension applied to tissue-coupling element 128 thus locks open loop 154 into a desired shape.

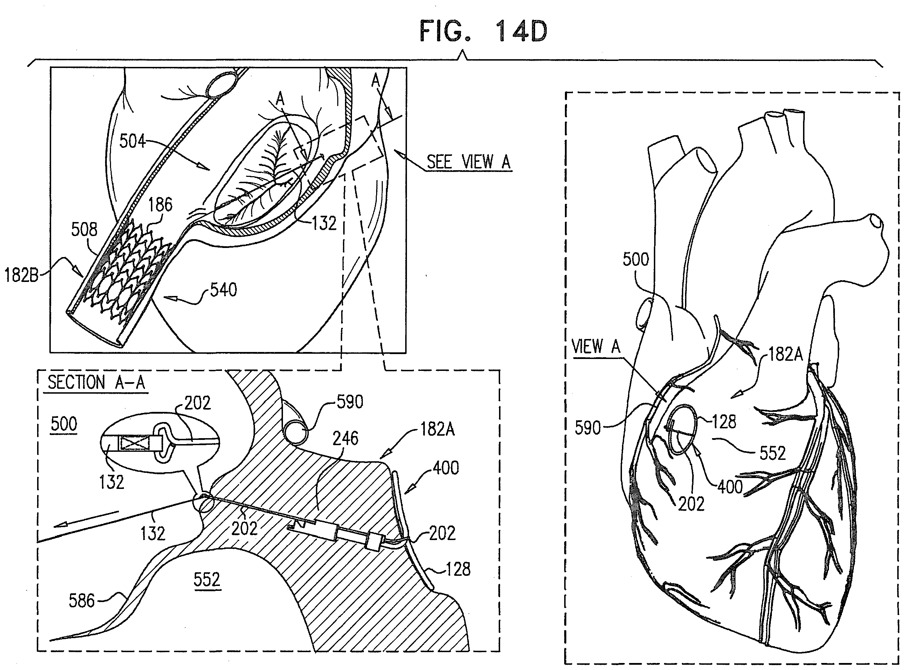

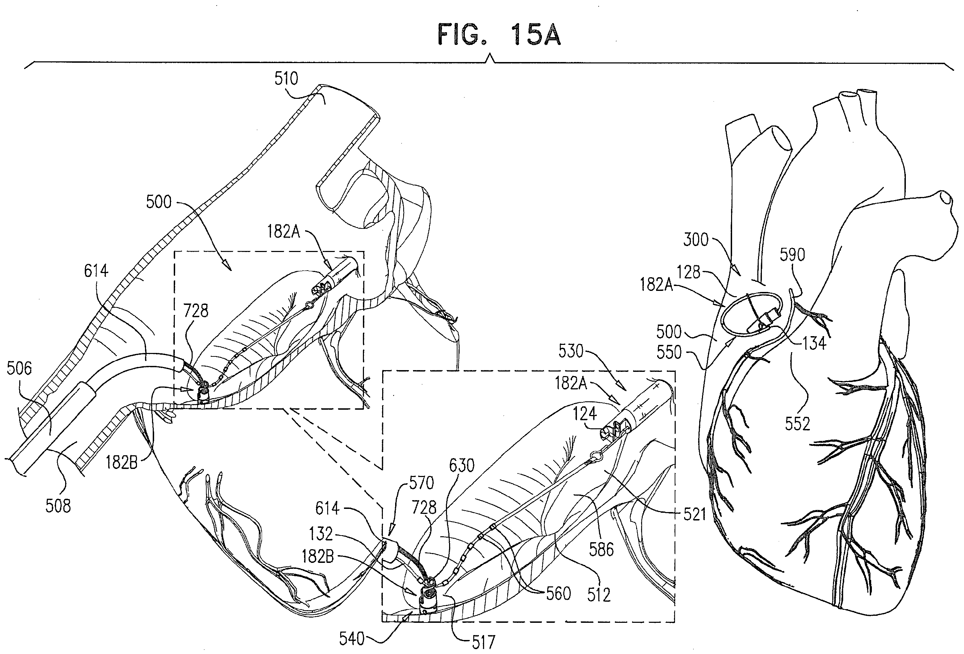

Locking stopper 270 limits the total load that can be applied to open loop 154 by flexible elongate tension member 202, thereby reducing excessive, unnecessary strain on open loop 154. For example, the first 1.5 to 5 N of force applied to flexible elongate tension member 202 may sufficiently deform open loop 154 and engage locking stopper 270. Additional load (tension) that is applied by flexible elongate tension member 202 pulls on the entire tissue anchor 400, and does not further increase the load applied across open loop 154 to site 206, and thus does not further compress the open loop. As described hereinbelow with reference to Figs. 14D and 15A-C, such tension may be applied to pull tissue anchor 400 closer to another tissue anchor, in order to facilitate repair of an atrioventricular valve of the subject, such as tricuspid valve 504.

These techniques thus allow the use of relatively flexible tissue-coupling element, in order to not generate too much outward force inside a delivery tube, which might make axial movement of the tissue-coupling element in the delivery tube difficult or impossible. The tissue-coupling element is tensioned upon delivery, thereby changing its shape and providing a strong tissue-coupling element that cannot unwind easily, and thus remains coupled to the tissue. In addition, minimizing the load on attachment site 206 provides a mechanical advantage that increases the durability of the device under higher loads.

As mentioned above, open loop 154 may have more than one turn and less than 1.5 turns, such as more than one turn, e.g., more than 1.01 turns (363.6 degrees), such as more than 1.02 turns (367.2 degrees), and/or less than 1.25 turns (450 degrees) (one turn equals 360 degrees). Providing open loop 154 with more than one turn, rather than exactly one turn or less than one turn, prevents crossing portion 212 from sliding down off of open loop 154 and onto anchor shaft 122 when tension is applied to crossing portion 212. Such sliding might result in crossing portion 212 cutting into tissue of the heart.

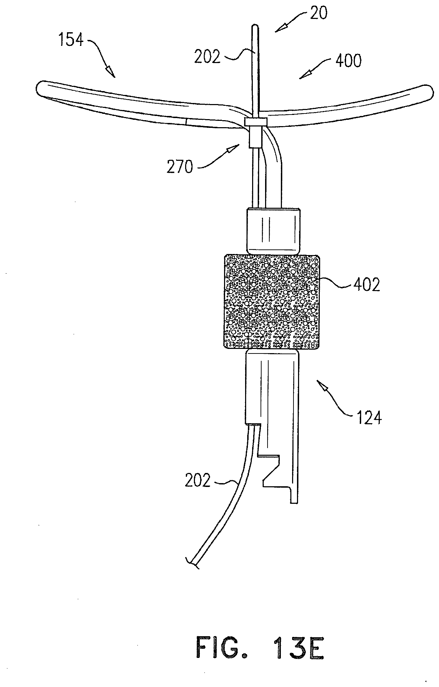

Reference is made to Fig. 13E, which is a schematic illustration of tissue anchor 400 comprising a sealing element 402. Sealing element 402 is similar in some respects to sealing element 190, described hereinabove with reference to Figs. 1A-D and 4A-B. Sealing element 402 is configured to form a blood-tight seal between a portion of anchor head 124 inside the heart chamber and wall 194 of the heart. For some applications, sealing element 402 comprises a compressible sponge. For some applications, an outer diameter of sealing element 402, when expanded, equals at least 1.5 times, e.g., at least 2 times, an inner diameter of deployment shaft 34 of deployment tool 30, described hereinabove with reference to Fig. 1A. For some applications, sealing element 402 is disposed on the narrower portion of anchor head 124 between two collars.

Reference is made to Figs. 1B-C, 3A-B, 4A-B, 5A-D, 7, 8, 9A-F, 10A-H, and 13A-E. For some applications, tissue anchor 20 is shaped so as to define a bend 480 at an interface between tissue-coupling element 128 and anchor shaft 122. Typically, bend 480 has an angle ε (epsilon) of between 45 and 135 degrees, e.g., between 60 and 120 degrees, such as between 85 and 105 degrees, e.g., 90 degrees, as labeled in Figs. 1C and 3B.

Reference is now made to Figs. 14A-D, which are schematic illustrations of a method for deploying tissue anchor system 248, described hereinabove with reference to Figs. 4A-B, for repairing tricuspid valve 504. In the particular method shown in these figures, first and second tissue anchors 182A and 182B of tissue anchor system 248 comprise first tissue anchor 400, described hereinabove with reference to Figs. 13A-E, and stent 186, described hereinabove with reference to Fig. 4B. The method may also be used to deploy other tissue anchors described herein, *mutatis mutandis,* including tissue anchor 300. Tissue anchor system 248 further comprises deployment tool 30, for deploying first tissue anchor 182A, and, typically, a second anchor delivery tool for deploying second tissue anchor 182B.

As shown in Fig. 14A, first anchor deployment tool 30 is advanced, during a transcatheter procedure (typically endovascularly, such as percutaneously). Also as shown in Fig. 14A, for applications in which deployment tool 30 is shaped so as to define sharp distal piercing tip 32, such as described hereinabove with reference to Fig. 1A, first anchor deployment tool 30 is advanced through the wall of the heart by advancing sharp distal piercing tip 32 of the tool through first implantation site 530. Successful passage through the wall is typically confirmed using imaging.

For applications in which tissue anchor 20 comprises tip 308, typically one of the configurations of deployment tool 30 described with reference to Figs. 2A-B or 6A-B is instead used; in these configurations, deployment tool 30(including deployment shaft 34) is not shaped so as define sharp distal piercing tip 32. Typically, guidewire 310 is first introduced through the wall of the heart using conventional transcatheter guidewire advancement techniques, and deployment tool 30, with tissue anchor 20 disposed therein, is deployed through the wall over the guidewire.

First implantation site 530 is shown as within 1 cm of the site on the annulus that circumferentially corresponds to circumferential middle 521 of anterior leaflet 586; alternative first implantation sites 530 are set forth hereinbelow in Table 1. For some applications, first implantation site 530 is within 10 mm, such as within 5 mm, of RCA 590.

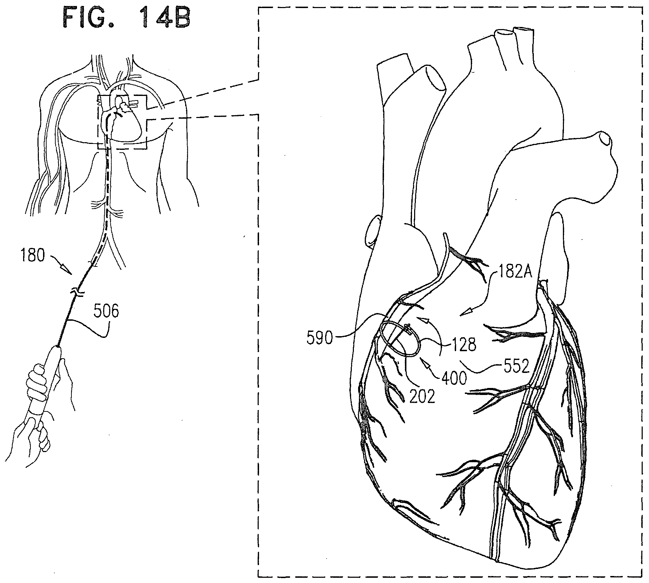

As shown in Fig. 14B, first tissue anchor 182A is partially released from first anchor deployment tool 30 such that tissue-coupling element 128 is unconstrained by first anchor deployment tool 30. The surgeon ascertains, typically using imaging, whether tissue-coupling element 128 overlies a coronary blood vessel, such as RCA 590. In the procedure shown in Fig. 14B, tissue-coupling element 128 does overlie a coronary blood vessel (RCA 590). For applications in which tissue anchor 20 comprises tip 308, once proper deployment of tissue-coupling element 128 is confirmed, guidewire 310 is withdrawn. (Guidewire 310 is shown in Figs. 3A-C for clarity of illustration; during the implantation procedure, the guidewire is typically withdrawn before the tissue anchor reaches.the configuration shown in these figures.)

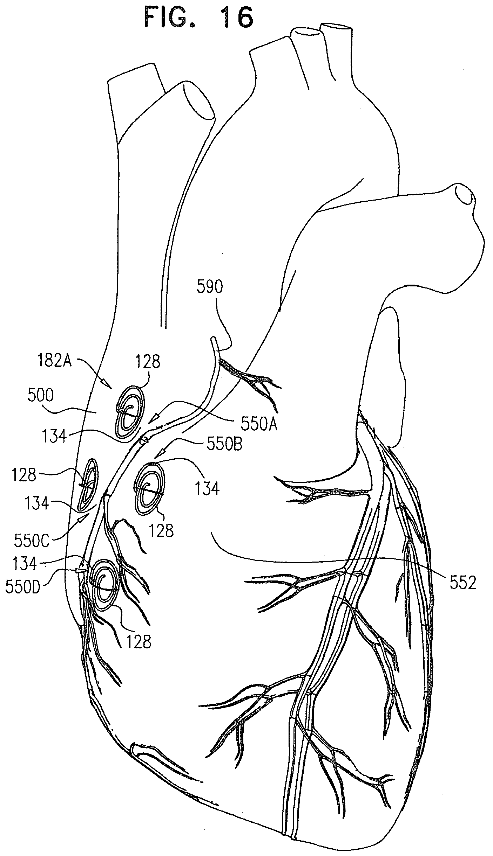

For some applications, such as shown in Figs. 14A-D, first anchor deployment tool 30, and first tissue anchor 182A, exit the heart at external exit site 550 on right atrium 500. Typically, in these applications, first tissue anchor 182A passes through an atrial portion of the annulus, or an edge of the annulus and the origin of the trabeculae carneae. For other applications, such as described hereinbelow with reference to Fig. 16, first anchor deployment tool 30, and first tissue anchor 182A, exit the heart at external exit site 550 on right ventricle 552. Typically, in these applications, first tissue anchor 182A passes under RCA 590 in the annulus and exits on the ventricular wall.

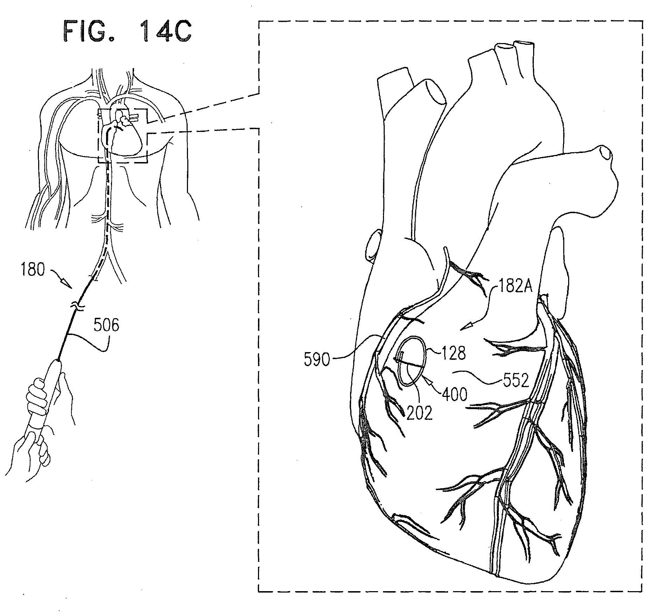

If tissue-coupling element 128 overlies a coronary blood vessel (e.g., RCA 590), the surgeon rotates first tissue anchor 182A (clockwise and/or counterclockwise, about central longitudinal axis 134) until tissue-coupling element 128 no longer overlies the coronary blood vessel, as shown in Fig. 14C. The rotation is typically performed by rotating anchor shaft 122. The surgeon brings tissue-coupling element 128 into contact with an external surface 534 of the heart, by proximally retracting first tissue anchor 182A.

Providing the tissue anchor (e.g., tissue anchor 400) with an elliptical shape (or paper clip shape) reduces the risk of contact with a sensitive anatomic structure, such as a blood vessel, e.g., the RCA.

After first tissue anchor 182A has been implanted at first implantation site 530, driver 201 is decoupled from the anchor head and deployment tool 30 is removed from the subject's body, typically leaving catheter 506 *in situ.*

As shown in Fig. 14D, second tissue anchor 182B is implanted in the subject at second implantation site 540. For example, as shown, second tissue anchor 182B may comprise stent 186, and second implantation site 540 may be inferior vena cava 508; an alternative second implantation site 540 is set forth hereinbelow in Table 1. Tension is applied to the one or more tethers 132 that couple the first tissue anchor 182A (e.g., flexible elongate tension member 202 thereof) to second tissue anchor 182B. Typically, the tension is applied without applying tension to anchor shaft 122. Application of such tension facilitates repair of an atrioventricular valve of the subject, such as tricuspid valve 504.

For some applications, second tissue anchor 182B is implanted in the subject, and first tissue anchor 182A is coupled to second tissue anchor 182B by the one or more tethers 132 using the techniques described for connecting first and second tissue-engaging elements 60a and 60b in US Patent Application Publication 2014/0114390 with reference to Figs. 34A-E thereof. For some applications, one of the one or more tethers 132 is fixed to one of (a) first tissue anchor 182A and (b) second tissue anchor 182B. For some applications, first and second tissue anchors 182A and 182B are implanted using techniques described in US Patent Application Publication 2012/0035712 with reference to Figs. 7A-D and/or Figs. 11A-B thereof.

The following Table 1 sets forth exemplary combinations of (a) anatomical markers for first implantation site 530, (b) second implantation site 540, and (c) external exit sites 550. These sites are listed by way of example and not limitation; the surgeon typically selects the exact sites based on the subject's individual needs and anatomy. Any appropriate location on the heart wall may be used. First implantation site 530 is located within 1 cm of the site on the annulus that circumferentially corresponds to the anatomical marker (i.e., is at the same angular location or "o'clock" as the respective anatomical marker). The direction of the 1 cm from the site on the annulus may be either circumferentially (i.e., clockwise or counterclockwise) around the annulus, up the wall of the right atrium above the annulus, or a combination of circumferentially around the annulus and up the wall of the atrium.

Typically, the surgeon uses the anatomical markers to find the exact location first implantation site 530, which is within 1 cm of the anatomical markers, as described above. For example, the commissures are easily detectable using imaging, and thus represent good anatomical markers. However, the commissures are not appropriate for implantation (because they are too delicate), so, in this example, the tissue anchors are implanted near the annulus, such as up the wall of the atrium, within 1 cm from the commissure.

**TABLE 1**

| | | |
|---|---|---|
| First implantation site 530 anatomical marker | Second implantation site 540 | External exit site 550 |
| Circumferential middle 521 of anterior leaflet 586 | Inferior vena cava 508 | Right atrium 500 (site 550A in Fig. 16) |
| An anteroposterior commissure 512 | Inferior vena cava 508 | Right atrium 500 (site 550B in Fig. 16) |
| Circumferential middle 521 of anterior leaflet 586 | Inferior vena cava 508 | Right ventricle 552 (site 550C in Fig. 16) |
| Anteroposterior commissure 512 | Inferior vena cava 508 | Right ventricle 552 (site 550D in Fig. 16) |
| A circumferential middle of a posterior leaflet | Superior vena cava 510 | Right ventricle 552 (site 550C in Fig. 16) |
| Anteroposterior commissure 512 | Superior vena cava 510 | Right ventricle 552 (site 550D in Fig. 16) |
| Circumferential middle 521 of anterior leaflet 586 | A coronary sinus | Right atrium 500 (site 550A in Fig. 16) |

Reference is now made to Figs. 15A-C, which are schematic illustrations of another method for deploying tissue anchor system 248 for repairing tricuspid valve 504. In the particular method shown in these figures, first tissue anchor 182A of tissue anchor system 248 comprises first tissue anchor 300, and second tissue anchor 182B of tissue anchor system 180 comprises helical tissue-coupling element 184, described hereinabove with reference to Fig. 4A. The method may also be used to deploy other tissue anchors described herein, including tissue anchor 200 or 400 of tissue anchor system 248 as the first tissue anchor, *mutatis mutandis.* Tissue anchor system 180 or tissue anchor system 248 further comprises deployment tool 30, for deploying first tissue anchor 182A, and, typically, a second anchor delivery tool 570 for deploying second tissue anchor 182B. For some applications, second anchor delivery tool 570 comprises a torque-delivery tool, such as described in PCT Publication WO 2015/193728. Tissue anchor system 248 allows first and second tissue anchors 182A and 182B to be delivered separately and connected afterwards *in situ.* This simplifies the procedure for the operator, and allows an approach from two or more different blood vessels such as transfemoral, transjugular, transradial or transapical approaches, which may provide simpler access to the anchoring point.

First tissue anchor 182A is implanted as described hereinabove with reference to Figs. 13A-D, as appropriate. As mentioned above, first implantation site 530 is shown as circumferential middle 521 of anterior leaflet 586; alternative first implantation sites 530 are set forth hereinbelow in Table 2. As mentioned with reference to Fig. 13C, after first tissue anchor 182A has been implanted at first implantation site 530, deployment tool 30 is removed from the subject's body, typically leaving catheter 506 *in situ.*

As shown in Fig. 15A, second tissue anchor 182B is implanted in the subject at second implantation site 540. For example, second tissue anchor 182B may comprise helical tissue-coupling element 184, described hereinabove with reference to Fig. 4A, and second implantation site 540 may be within 1 cm of a site on the annulus that circumferentially corresponds to a septoposterior commissure 517; alternative second implantation sites 540 are set forth hereinbelow in Table 2. For some applications, the one or more tethers 132 comprise a single tether 132. For some applications, tether 132 defines a plurality of securement protrusions 560 spaced at intervals along tether 132, which protrusions serve as the friction-enhancing features. For some applications, as shown, protrusions 560 comprise respective cylinders on tether 132.

For some applications, outside the subject's body, the surgeon threads a free end of tether 132 through a lateral opening 582 of an outer tether-securing element 580 of second tissue anchor 182B, and then through a lumen of a delivery tube 614. Tether 132 thus connects first and second tissue anchors 182A and 182B.

For some applications, as shown in Fig. 15A, second tissue anchor 182B is implanted at second implantation site 540 using a torque-delivery cable 728 of the torque-delivery tool, as described in above-mentioned PCT Publication WO 2015/193728. Second tissue anchor 182B and torque-delivery cable 728 are introduced over tether 132 and through delivery tube 614, which itself is advanced through catheter 506. A tetherlocking mechanism of second tissue anchor 182B is introduced in an unlocked state in which sliding of tether 132 through a lateral opening of second tissue anchor 182B is not inhibited. Second tissue anchor 182B is implanted at second implantation site 540 by rotating torque-delivery cable 728 (including a torque-delivery head).

The size of the tricuspid valve orifice is reduced by tensioning tether 132, so as to reduce regurgitation. Such tensioning may be performed by proximally pulling on the free end of tether 132, such that a portion of tether 132 is pulled through lateral opening 582 of second tissue anchor 182B. This tension can be applied remotely, i.e., via catheter 506. Application of such tension facilitates repair of an atrioventricular valve of the subject, such as tricuspid valve 504.

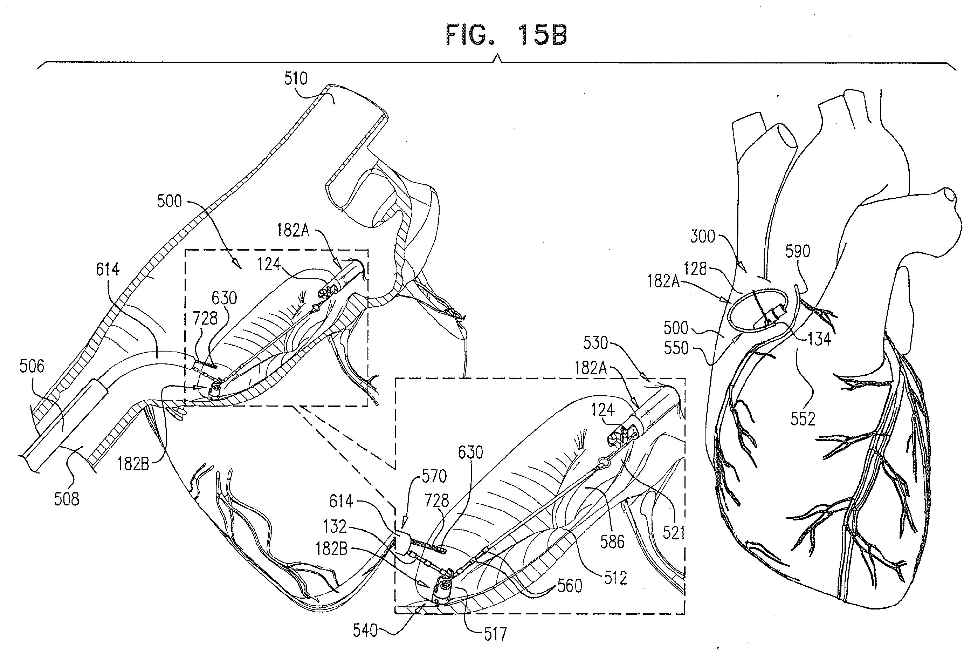

As shown in Fig. 15B, once the tension has been applied, torque-delivery cable 728 (including the torque-delivery head) is decoupled from second tissue anchor 182B, such as by removing a locking wire. As a result, a spring expands and presses tether 132 against an outer tether-securing element 780, both of which are described in above-mentioned PCT Publication WO 2015/193728. This pressing transitions the tetherlocking mechanism to a locked state, in which state the sliding of tether 132 through the second tissue anchor 182B is inhibited. Such locking maintains the distance and tension between second tissue anchor 182B and first tissue anchor 182B.

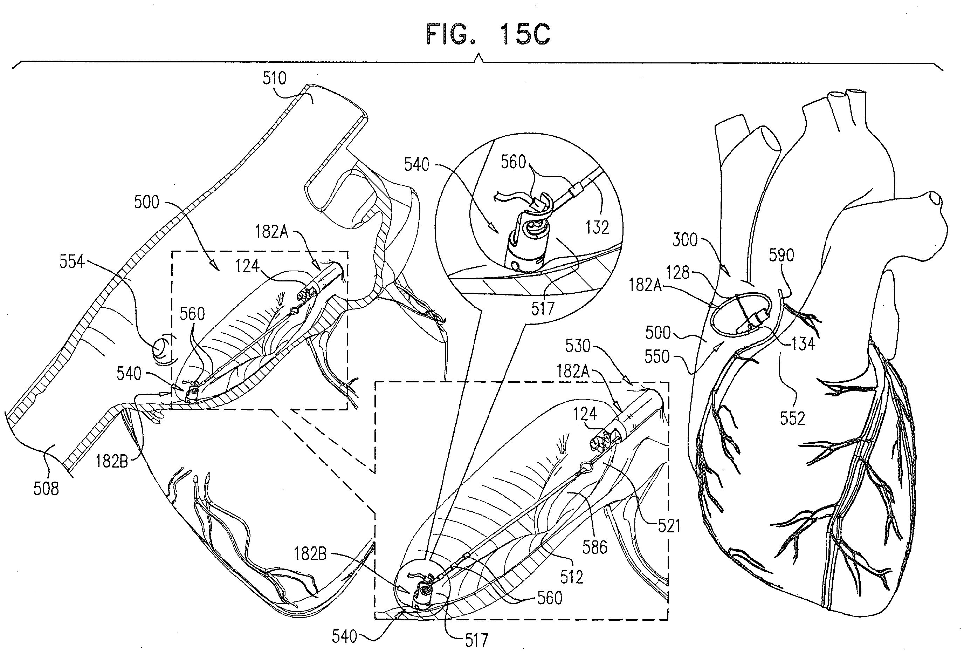

As shown in Fig. 15C, after tether 132 has been tensioned, an excess portion of tether 132 remains free in the right atrium. It is generally undesirable to leave this excess portion free to move around in the atrium. For some applications, the excess portion of tether 132 is cut and removed from the atrium, using a cutting tool, such as thoracoscopic scissors, as known in the art. Further alternatively, for some applications, the excess portion is secured in a desired disposition in the vasculature of the right atrium, such as in inferior vena cava 508, superior vena cava 510, or a coronary sinus.

The following Table 2 sets forth exemplary combinations of (a) anatomical markers for first implantation site 530, (b) anatomical markers for second implantation site 540, and (c) external exit sites 550. These sites are listed by way of example and not limitation; the surgeon typically selects the exact sites based on the subject's individual needs and anatomy. Each of first and second implantation sites 530 and 540 is located within 1 cm of the site on the annulus that circumferentially corresponds to the respective anatomical marker (i.e., is at the same angular location or "o'clock" as the respective anatomical marker). The direction of the 1 cm from the site on the annulus may be either circumferentially (i.e., clockwise or counterclockwise) around the annulus, up the wall of the right atrium above the annulus, or a combination of circumferentially around the annulus and up the wall of the atrium. For example, as shown in Fig. 15C, septoposterior commissure 517 is near, but not on, the annulus, and second tissue anchor 182B is shown implanted at second implantation site 540, which is at the site on the annulus that circumferentially corresponds to this commissure. Second implantation site 540 could also be up to 1 cm clockwise or counterclockwise around the annulus from this site on the annulus, up to 1 cm up the wall of the atrium, or a combination of these two directions.

Typically, the surgeon uses the anatomical markers to find the exact locations of first and second implantation sites 530 and 540, which are within 1 cm of the anatomical markers, as described above. For example, the commissures are easily detectable using imaging, and thus represent good anatomical markers. However, the commissures are not appropriate for implantation (because they are too delicate), so, in this example, second tissue anchor 182B is implanted on the annulus or up the wall of the atrium, within 1 cm from the commissure.

**TABLE 2**

| | | |
|---|---|---|
| First implantation site 530 anatomical marker | Second implantation site 540 anatomical marker | External exit site 550 |
| Circumferential middle 521 of anterior leaflet 586 | Septoposterior commissure 517 | Right atrium 500 |
| Anteroposterior commissure 512 | Septoposterior commissure 517 | Right atrium 500 |
| Circumferential middle 521 of anterior leaflet 586 | Septoposterior commissure 517 | Right ventricle 552 |
| Anteroposterior commissure 512 | Septoposterior commissure 517 | Right ventricle 552 |
| Anteroposterior commissure 512 | a coronary sinus ostium 554 (labeled in Fig. 15C) | Right ventricle 552 |

Reference is now made to Fig. 16, which is a schematic illustration of several external exit sites 550. External exit sites 550 are typically within 10 mm, such as 5 mm, of RCA 590 or branches from the RCA such as the posterior descending artery (PDA) or veins of the right ventricle. External exit sites 550A and 550C are on right atrium 500, and external exit sites 550B and 550D are on right ventricle 552.

For some applications, both first and second tissue anchors 182A and 182B comprise respective tissue anchors 20 (tissue anchors 200, 300, 340, 350, 430, 370, 400, 420, 470, or 490, or a combination of two different ones of these tissue anchors). For some applications, first tissue anchor 182A is implanted at an implantation site located with 1 cm of the site on the annulus that circumferentially corresponds to an anatomical marker between circumferential middle 521 of anterior leaflet 586 and anteroposterior commissure 512, inclusive. Alternatively or additionally, for some applications, second tissue anchor 182B is implanted at an implantation site located with 1 cm of the site on the annulus that circumferentially corresponds to an anatomical marker between a circumferential middle of a posterior leaflet and septoposterior commissure 517, inclusive.

Further alternatively or additionally, for some applications, second tissue anchor 182B is implanted at an implantation site located above the triangle of Koch, through the septal muscle into the left atrium above the level of the mitral valve. The off-centeredness of tissue anchor 20 allows the tissue-coupling element to be rotated during implantation so as to avoid contact with the mitral valve if the anchor enters the left atrium lower than expected. For some of these applications, first tissue anchor 182A comprises a stent, such as described hereinabove, which may be connected to second tissue anchor 182B by one or more tethers, at least one of which passes through a pulley, such as described in PCT Publication WO 2015/063580. Alternatively, the tissue anchors are implanted and coupled to one another under tension using the techniques described hereinabove with reference to Fig. 15B, *mutatis mutandis.*

Reference is made to Figs. 1A-16. For some applications, apparatus is provided for delivery in a constrained state within deployment tool 30, the apparatus comprising tissue anchor 20, which comprises (a) anchor shaft 122, and (b) tissue-coupling element 128, which comprises wire 150. When tissue anchor 20 is unconstrained by deployment tool 30:
- anchor shaft 122 has central longitudinal axis 134,
- for applications in which tissue-coupling element 128 does not comprise tip 308, wire 150 of tissue-coupling element 128 is shaped as open loop 154 having more than one turn (optionally, around a center point 162), and (b) for applications in which tissue-coupling element 128 comprises tip 308, tissue-coupling element 128 is shaped as open loop 354 having more than one turn (optionally, around center point 162), and
- wire 150 extends from distal end 130 of anchor shaft 122 at a radially-outer end 164 of open loop 154 or 354, as the case may be.

For some applications, a method is provided that comprises (a) providing tissue anchor 20 having the characteristics described immediately above; (b) introducing, during a transcatheter procedure, tissue anchor 20 into a cardiac chamber of a heart of a subject, while tissue-coupling element 128 is constrained by deployment tool 30; (c) delivering tissue-coupling element 128 through a wall of the heart; and (d) at least partially releasing tissue anchor 20 from deployment tool 30.

Although the techniques described herein have been described as being used to remodel the tricuspid valve, these techniques may also be used to remodel the mitral valve, *mutatis mutandis.* In addition, the tissue anchors described herein may be implanted on the surface of any wall of the heart or other organ where tension is to be applied, and rotationally repositioned to avoid obstructions of anatomic structures such as blood vessels or conduction systems, or pre-existing implants.

As used in the present application, including in the claims, when a range of values is specified using the word "between," the range includes the endpoint values.

The following documents, which are assigned to the assignee of the present application are referenced herewith:
- US Patent 8,475,525 to Maisano et al.;
- US Patent 8,961,596 to Maisano et al.;
- US Patent 8,961,594 to Maisano et al.;
- International Application PCT/IL2011/000064, filed January 20, 2011, which published as PCT Publication WO 2011/089601, and US Application 13/574,088 in the national stage thereof, which published as US Patent Application Publication 2013/0046380;
- US Application 13/553,081, filed July 19, 2012, which published as US Patent Application Publication 2013/0018459;
- International Application PCT/IL2012/000282, filed July 19, 2012, which published as PCT Publication WO 2013/011502;
- US Provisional Application 61/750,427, filed January 9, 2013;
- US Provisional Application 61/783,224, filed March 14, 2013;
- International Application PCT/IL2013/050470, filed May 30, 2013, which published as PCT Publication WO 2013/179295;
- US Provisional Application 61/897,491, filed October 30, 2013;
- US Provisional Application 61/897,509, filed October 30, 2013;
- US Application 14/143,355, filed December 30, 2013, which published as US Patent Application Publication 2014/0114390;
- International Application PCT/IL2014/050027, filed January 9, 2014, which published as PCT Publication WO 2014/108903;
- International Application PCT/IL2014/050233, filed March 9, 2014, which published as PCT Publication WO 2014/141239;
- US Provisional Application 62/014,397, filed June 19, 2014;
- International Application PCT/IB2014/002351, filed October 28, 2014, which published as PCT Publication WO 2015/063580;
- US Application 14/525,668, filed October 28, 2014, which published as US Patent Application Publication 2015/0119936;
- US Provisional Application 62/086,269, filed December 2, 2014;
- US Provisional Application 62/131,636, filed March 11, 2015;
- US Provisional Application 62/167,660, filed May 28, 2015; and
- International Application PCT/IB2015/001196, filed June 14, 2015, which published as PCT Publication WO 2015/193728.

## Claims

1. Apparatus for delivery through a cardiac tissue wall in a constrained state within a deployment tool (30), the apparatus comprising a tissue anchor (290), which comprises:
an anchor shaft (122) having a central longitudinal axis (134);
a tissue-coupling element (128), configured to have a predetermined shape when unconstrained by the deployment tool and automatically transition to the predetermined shape when released from being constrained by the deployment tool (30), and which (a) extends from a distal end (130) of the anchor shaft (122), and (b) comprises a wire (150), wherein when the tissue anchor (290) is unconstrained by the deployment tool (30): (a) the wire (150) is shaped as an open shape (291), and (b) if the tissue-coupling element (128) were to be projected onto a plane (136) that is perpendicular to the central longitudinal axis (134), the projection of the open shape (291) would surround between 170 and 355 degrees of a point (292) in the plane (136); and
a flexible elongate tension member (202), which includes (a) a distal portion (204) that is fixed to a site (206) on the wire (150), (b) a proximal portion (208), which has a longitudinal segment (209) that runs alongside at least a portion (210) of the anchor shaft (122), and (c) a crossing portion (212), which (i) is disposed between the distal and the proximal portions (204, 208) along the flexible elongate tension member (202), and (ii) crosses at least a portion of the open shape (291) when the tissue anchor (290) is unconstrained by the deployment tool (30),
wherein the tissue anchor (290) is configured to allow relative axial motion between the at least a portion (210) of the anchor shaft (122) and the longitudinal segment (209) of the proximal portion (208) of the flexible elongate tension member (202) when the tissue anchor (290) is unconstrained by the deployment tool (30),
wherein the point (292) falls on a projection onto the plane (136) of a line segment that terminates at (a) the site (206) on the wire (150) and (b) a proximal end (382) of the wire (150) when the tissue anchor (290) is unconstrained by the deployment tool (30).

2. The apparatus according to claim 1,
wherein the tissue anchor (290) comprises an anchor head (124) connected to a proximal portion (208) of the anchor shaft (122),
wherein the anchor head (124) is shaped so as to define a passage (272) in which the proximal portion (208) of the flexible elongate tension member (202) is slidably disposed,
wherein the flexible elongate tension member (202) comprises a locking stopper (270), which is axially fixed to the proximal portion (208) or the crossing portion (212) of the flexible elongate tension member (202), and
wherein the locking stopper (270) and the passage (272) are sized and shaped such that the size and shape of the passage (272) prevent proximal movement of the locking stopper (270) past the passage (272).

3. The apparatus according to claim 1, wherein the open shape (291) is shaped as a portion of a circle or a portion of an ellipse when the tissue anchor (290) is unconstrained by the deployment tool (30).

4. The apparatus according to claim 1, wherein at least a portion of the tissue-coupling element (128) is curved.

5. The apparatus according to claim 1, wherein the site (206) on the wire (150) is located within 7 mm of a distal end (294) of the open shape (291).

6. The apparatus according to claim 1, wherein the site (206) on the wire (150) is at a distal end (294) of the wire (150).

7. The apparatus according to claim 6, wherein the wire (150) is shaped so as to define a channel, through which a portion of the flexible elongate tension member (202) passes and exits the wire (150) at the distal end (294) of the wire (150).

8. The apparatus according to claim 1, wherein a radius of the flexible elongate tension member (202) is less than a radius of the wire (150).

9. The apparatus according to claim 8, wherein the radius of the flexible elongate tension member (202) is less than 50% of the radius of the wire (150).

10. The apparatus according to any one of claims 1-9, wherein, when the tissue anchor (290) is unconstrained by the deployment tool (30), (a) a line segment that terminates at (i) the site (206) on the wire (150) and (ii) a proximal end of the tissue-coupling element (128) has a total length that equals a percentage of (b) a total length of the tissue-coupling element (128), measured along the tissue-coupling element (128), the percentage between 25% and 75%.

11. The apparatus according to any one of claims 1-9, wherein, when the tissue anchor (290) is unconstrained by the deployment tool (30):
a greatest longitudinal dimension of the open shape (291), measured in parallel to a central longitudinal axis (134) of the anchor shaft (122), is between 0.25 and 5 mm, and
a greatest lateral dimension of the open shape (291), measured perpendicular to the central longitudinal axis (134), is between 4 and 20 mm.

12. The apparatus according to claim 1, wherein the tissue anchor (290) is shaped so as to define a bend (480) at an interface between the tissue-coupling element (128) and the anchor shaft (122).

13. The apparatus according to claim 12, wherein the bend (480) has an angle of between 45 and 135 degrees.

14. The apparatus according to claim 1, wherein the tissue-coupling element (128) comprises a tip (308), which is fixed to a distal end (294) of the wire (150), and has, at a widest longitudinal site (312) along the tip (308), a greatest tip outer cross-sectional area that equals at least 150% of an average wire cross-sectional area of the wire (150).

15. The apparatus according to claim 1, wherein the anchor shaft (122) and the tissue-coupling element (128) are integral to one another and together comprise the wire (150).

## Patentansprüche

1. Vorrichtung zum Zuführen durch eine Herzgewebewand in einem eingeschränkten Zustand innerhalb eines Entfaltungswerkzeugs (30), die Vorrichtung umfassend einen Gewebeanker (290), der Folgendes umfasst:
einen Ankerschaft (122), der eine mittlere Längsachse (134) aufweist;
ein Gewebekopplungselement (128), das konfiguriert ist, um eine vorbestimmte Form aufzuweisen, wenn es durch das Entfaltungswerkzeug nicht eingeschränkt ist, und automatisch in die vorbestimmte Form übergeht, wenn es von der Einschränkung durch das Entfaltungswerkzeug (30) befreit wird, und das (a) sich von einem distalen Ende (130) des Ankerschafts (122) erstreckt, und (b) einen Draht (150) umfasst, wobei, wenn der Gewebeanker (290) durch das Entfaltungswerkzeug (30) nicht eingeschränkt ist: (a) der Draht (150) als eine offene Form (291) geformt ist, und (b) wenn das Gewebekopplungselement (128) auf eine Ebene (136) projiziert werden würde, die senkrecht zu der mittleren Längsachse (134) ist, die Projektion der offenen Form (291) zwischen 170 und 355 Grad eines Punkts (292) in der Ebene (136) umschließen würde; und
ein flexibles längliches Zugelement (202), das (a) einen distalen Abschnitt (204), der an einer Stelle (206) auf dem Draht (150) befestigt ist, (b) einen proximalen Abschnitt (208), der ein Längssegment (209) aufweist, das entlang mindestens eines Abschnitts (210) des Ankerschafts (122) verläuft, und (c) einen Kreuzungsabschnitt (212), der (i) zwischen dem distalen und dem proximalen Abschnitt (204, 208) entlang des flexiblen länglichen Zugelements (202) angeordnet ist und (ii) mindestens einen Abschnitt der offenen Form (291) kreuzt, wenn der Gewebeanker (290) durch das Entfaltungswerkzeug (30) nicht eingeschränkt ist, beinhaltet,
wobei der Gewebeanker (290) konfiguriert ist, um eine relative axiale Bewegung zwischen dem mindestens einen Abschnitt (210) des Ankerschafts (122) und dem Längssegment (209) des proximalen Abschnitts (208) des flexiblen länglichen Zugelements (202) zuzulassen, wenn der Gewebeanker (290) durch das Entfaltungswerkzeug (30) nicht eingeschränkt ist, wobei
der Punkt (292) auf eine Projektion auf die Ebene (136) eines Liniensegments fällt, das an (a) der Stelle (206) auf dem Draht (150) und (b) einem proximalen Ende (382) des Drahts (150) endet, wenn der Gewebeanker (290) durch das Entfaltungswerkzeug (30) nicht eingeschränkt ist.

2. Vorrichtung nach Anspruch 1,
wobei der Gewebeanker (290) einen Ankerkopf (124) umfasst, der mit einem proximalen Abschnitt (208) des Ankerschafts (122) verbunden ist,
wobei der Ankerkopf (124) geformt ist, um einen Durchgang (272) zu definieren, in dem der proximale Abschnitt (208) des flexiblen, länglichen Zugelements (202) gleitend angeordnet ist,
wobei das flexible längliche Zugelement (202) einen Verriegelungsanschlag (270) umfasst, der axial an dem proximalen Abschnitt (208) oder dem Kreuzungsabschnitt (212) des flexiblen länglichen Zugelements (202) befestigt ist, und
wobei der Verriegelungsanschlag (270) und der Durchgang (272) bemessen und geformt sind, sodass die Größe und Form des Durchgangs (272) eine proximale Bewegung des Verriegelungsanschlags (270) an dem Durchgang (272) vorbei verhindern.

3. Vorrichtung nach Anspruch 1, wobei die offene Form (291) als ein Abschnitt eines Kreises oder ein Abschnitt einer Ellipse geformt ist, wenn der Gewebeanker (290) durch das Entfaltungswerkzeug (30) nicht eingeschränkt ist.

4. Vorrichtung nach Anspruch 1, wobei mindestens ein Abschnitt des Gewebekopplungselements (128) gekrümmt ist.

5. Vorrichtung nach Anspruch 1, wobei sich die Stelle (206) an dem Draht (150) innerhalb von 7 mm von einem distalen Ende (294) der offenen Form (291) befindet.

6. Vorrichtung nach Anspruch 1, wobei sich die Stelle (206) an dem Draht (150) an einem distalen Ende (294) des Drahts (150) befindet.

7. Vorrichtung nach Anspruch 6, wobei der Draht (150) geformt ist, um einen Kanal zu definieren, durch den ein Abschnitt des flexiblen, länglichen Zugelements (202) verläuft und an dem distalen Ende (294) des Drahts (150) aus dem Draht (150) austritt.

8. Vorrichtung nach Anspruch 1, wobei ein Radius des flexiblen, länglichen Zugelements (202) weniger ist als ein Radius des Drahts (150).

9. Vorrichtung nach Anspruch 8, wobei der Radius des flexiblen, länglichen Zugelements (202) weniger als 50 % des Radius des Drahts (150) ist.

10. Vorrichtung nach einem der Ansprüche 1-9, wobei, wenn der Gewebeanker (290) durch das Entfaltungswerkzeug (30) nicht eingeschränkt ist, (a) ein Liniensegment, das an (i) der Stelle (206) an dem Draht (150) und (ii) einem proximalen Ende des Gewebekopplungselements (128) endet, eine Gesamtlänge aufweist, die einem Prozentsatz von (b) einer Gesamtlänge des Gewebekopplungselements (128), gemessen entlang des Gewebekopplungselements (128), entspricht, wobei der Prozentsatz zwischen 25 % und 75 % ist.

11. Vorrichtung nach einem der Ansprüche 1 bis 9, wobei, wenn der Gewebeanker (290) durch das Entfaltungswerkzeug (30) nicht eingeschränkt ist:
eine größte Längsabmessung der offenen Form (291), gemessen parallel zu einer mittleren Längsachse (134) des Ankerschafts (122), zwischen 0,25 und 5 mm ist, und
eine größte seitliche Abmessung der offenen Form (291), gemessen senkrecht zur mittleren Längsachse (134), zwischen 4 und 20 mm ist.

12. Vorrichtung nach Anspruch 1, wobei der Gewebeanker (290) geformt ist, um eine Biegung (480) an einer Schnittstelle zwischen dem Gewebekopplungselement (128) und dem Ankerschaft (122) zu definieren.

13. Vorrichtung nach Anspruch 12, wobei die Biegung (480) einen Winkel zwischen 45 und 135 Grad aufweist.

14. Vorrichtung nach Anspruch 1, wobei das Gewebekopplungselement (128) eine Spitze (308) umfasst, die an einem distalen Ende (294) des Drahts (150) befestigt ist und an einer breitesten Längsstelle (312) entlang der Spitze (308) eine größte äußere Spitzenquerschnittsfläche aufweist, die mindestens gleich wie 150 % einer durchschnittlichen Drahtquerschnittsfläche des Drahts (150) ist.

15. Vorrichtung nach Anspruch 1, wobei der Ankerschaft (122) und das Gewebekopplungselement (128) miteinander verbunden sind und zusammen den Draht (150) umfassen.

## Revendications

1. Appareil permettant une administration à travers une paroi de tissu cardiaque dans un état contraint à l'intérieur d'un outil de déploiement (30), l'appareil comprenant un ancrage de tissu (290), qui comprend :
une tige d'ancrage (122) présentant un axe longitudinal central (134) ;
un élément de couplage de tissu (128), conçu pour avoir une forme prédéfinie lorsqu'il n'est pas contraint par l'outil de déploiement et passer automatiquement à la forme prédéfinie lorsqu'il est libéré d'être contraint par l'outil de déploiement (30), et qui (a) s'étend à partir d'une extrémité distale (130) de la tige d'ancrage (122), et (b) comprend un fil (150), lorsque l'ancrage de tissu (290) n'est pas contraint par l'outil de déploiement (30) : (a) le fil (150) étant formé sous la forme d'une forme ouverte (291), et (b) si l'élément de couplage de tissu (128) devait être projeté sur un plan (136) qui est perpendiculaire à l'axe longitudinal central (134), la projection de la forme ouverte (291) entourerait entre 170 et 355 degrés d'un point (292) dans le plan (136) ; et
un élément de tension allongé flexible (202) qui comprend (a) une partie distale (204) qui est fixée à un site (206) sur le fil (150), (b) une partie proximale (208) qui possède un segment longitudinal (209) qui longe au moins une partie (210) de la tige d'ancrage (122) et (c) une partie de croisement (212), qui (i) est disposée entre les parties distale et proximale (204, 208) le long de l'élément de tension allongé flexible (202), et (ii) croise au moins une partie de la forme ouverte (291) lorsque l'ancrage de tissu (290) n'est pas contraint par l'outil de déploiement (30),
ledit ancrage de tissu (290) étant conçu pour permettre un mouvement axial relatif entre ladite au moins une partie (210) de la tige d'ancrage (122) et le segment longitudinal (209) de la partie proximale (208) de l'élément de tension allongé flexible (202) lorsque l'ancrage de tissu (290) n'est pas contraint par l'outil de déploiement (30),
ledit point (292) tombant sur une projection sur le plan (136) d'un segment de droite qui se termine au niveau (a) du site (206) sur le fil (150) et (b) d'une extrémité proximale (382) du fil (150) lorsque l'ancrage de tissu (290) n'est pas contraint par l'outil de déploiement (30).

2. Appareil selon la revendication 1,
ledit ancrage de tissu (290) comprenant une tête d'ancrage (124) reliée à une partie proximale (208) de la tige d'ancrage (122),
ladite tête d'ancrage (124) étant formée de façon à définir un passage (272) dans lequel la partie proximale (208) de l'élément de tension allongé flexible (202) est disposée de manière coulissante,
ledit élément de tension allongé flexible (202) comprenant une butée de blocage (270) qui est fixée axialement à la partie proximale (208) ou à la partie de croisement (212) de l'élément de tension allongé flexible (202), et
ladite butée de blocage (270) et ledit passage (272) étant dimensionnés et formés de sorte que la taille et la forme du passage (272) empêchent un déplacement proximal de la butée de blocage (270) au-delà du passage (272).

3. Appareil selon la revendication 1, ladite forme ouverte (291) étant formée sous la forme d'une partie de cercle ou d'une partie d'ellipse lorsque l'ancrage de tissu (290) n'est pas contraint par l'outil de déploiement (30).

4. Appareil selon la revendication 1, au moins une partie de l'élément de couplage de tissu (128) étant incurvée.

5. Appareil selon la revendication 1, ledit site (206) sur le fil (150) étant situé à moins de 7 mm d'une extrémité distale (294) de la forme ouverte (291).

6. Appareil selon la revendication 1, ledit site (206) sur le fil (150) étant à une extrémité distale (294) du fil (150).

7. Appareil selon la revendication 6, ledit fil (150) étant formé de manière à définir un canal, à travers lequel une partie de l'élément de tension allongé flexible (202) passe et sort du fil (150) au niveau de l'extrémité distale (294) du fil (150).

8. Appareil selon la revendication 1, le rayon de l'élément de tension allongé flexible (202) étant inférieur au rayon du fil (150).

9. Appareil selon la revendication 8, ledit rayon de l'élément de tension allongé flexible (202) représentant moins de 50 % du rayon du fil (150).

10. Appareil selon l'une quelconque des revendications 1 à 9, lorsque l'ancrage de tissu (290) n'est pas contraint par l'outil de déploiement (30), (a) un segment de droite qui se termine au niveau (i) du site (206) sur le fil (150) et (ii) d'une extrémité proximale de l'élément de couplage de tissu (128) ayant une longueur totale qui est égale à un pourcentage de (b) la longueur totale de l'élément de couplage de tissu (128), mesurée le long de l'élément de couplage de tissu (128), le pourcentage étant compris entre 25 % et 75 %.

11. Appareil selon l'une quelconque des revendications 1 à 9, lorsque l'ancrage de tissu (290) n'est pas contraint par l'outil de déploiement (30) :
la dimension longitudinale la plus grande de la forme ouverte (291), mesurée parallèlement à un axe longitudinal central (134) de la tige d'ancrage (122), étant comprise entre 0,25 et 5 mm, et
la dimension latérale la plus grande de la forme ouverte (291), mesurée perpendiculairement à l'axe longitudinal central (134), étant comprise entre 4 et 20 mm.

12. Appareil selon la revendication 1, l'ancrage de tissu (290) étant formé de manière à définir un coude (480) au niveau d'une interface entre l'élément de couplage de tissu (128) et la tige d'ancrage (122).

13. Appareil selon la revendication 12, ledit coude (480) ayant un angle compris entre 45 et 135 degrés.

14. Appareil selon la revendication 1, ledit élément de couplage de tissu (128) comprenant une pointe (308) qui est fixée à une extrémité distale (294) du fil (150), et présentant, au niveau du site longitudinal le plus large (312) le long de la pointe (308), l'aire de section transversale externe de pointe la plus grande qui est égale à au moins 150 % de l'aire de section transversale de fil moyenne du fil (150).

15. Appareil selon la revendication 1, ladite tige d'ancrage (122) et ledit élément de couplage de tissu (128) étant intégrés l'un à l'autre et comprenant ensemble le fil (150).
